(19) Europäisches Patentamt
European Patent Office
Office européen des brevets

(11) Veröffentlichungsnummer: **0 281 842 B1**

(12) **EUROPÄISCHE PATENTSCHRIFT**

(45) Veröffentlichungstag der Patentschrift: **17.07.91**

(21) Anmeldenummer: **88102676.9**

(22) Anmeldetag: **24.02.88**

Die Akte enthält technische Angaben, die nach dem Eingang der Anmeldung eingereicht wurden und die nicht in dieser Patentschrift enthalten sind.

(51) Int. Cl.5: **C07D 405/06**, C07D 413/06, C07D 411/06, C07D 317/72, C07D 327/04, C07D 417/12, C07D 411/14, A01N 43/28, A01N 43/30

(54) **Aminomethylheterocyclen.**

(30) Priorität: **07.03.87 DE 3707364**
**21.10.87 DE 3735555**

(43) Veröffentlichungstag der Anmeldung:
**14.09.88 Patentblatt 88/37**

(45) Bekanntmachung des Hinweises auf die Patenterteilung:
**17.07.91 Patentblatt 91/29**

(84) Benannte Vertragsstaaten:
**BE CH DE ES FR GB IT LI NL**

(56) Entgegenhaltungen:
**EP-A- 0 097 822**
**DE-A- 1 965 321**

**CHEMICAL ABSTRACTS, Band 90, Nr. 9, 26. Februar 1979, Columbus, Ohio, USA; WOLINSKI J. et al.: "Search for anticholinergic compounds. VII. Synthesis of 2-aminomethyl-, 4-aminomethyl-, and 2,4-bis(aminomethyl)-1,3-dioxolanes", Seite 508, Spalte 1, Zusammenfassung Nr. 72 094q**

(73) Patentinhaber: **BAYER AG**

**W-5090 Leverkusen 1 Bayerwerk(DE)**

(72) Erfinder: **Krämer, Wolfgang, Dr.**
**Rosenkranz 25**
**W-5093 Burscheid 2(DE)**
Erfinder: **Weissmüller, Joachim, Dr.**
**Carl-Langhans-Strasse 53**
**W-4019 Monheim(DE)**
Erfinder: **Berg, Dieter, Dr.**
**Gellertweg 27**
**W-5600 Wuppertal 1(DE)**
Erfinder: **Dutzmann, Stefan, Dr.**
**Leinenweberweg 33**
**W-4000 Düsseldorf 13(DE)**

**EP 0 281 842 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

CHEMICAL ABSTRACTS, Band 88, Nr. 1, 2. Januar 1978, Columbus, Ohio, USA; KUTSUMA T. et al.: "1-(1,3-Dioxolan-4-ylmethyl)piperidinol derivatives", Seite 586, Spalte 1, Zusammenfassung Nr. 6 859a

CHEMICAL ABSTRACTS, Band 87, Nr. 21, 21. November 1977, Columbus, Ohio, USA; KUTSUMA T. et al.: "1-(1,3-Dioxolan-4-ylmethyl)piperidinol derivatives", Seite 575, Spalte 2, Zusammenfassung Nr. 168 008d

CHEMICAL ABSTRACTS, Band 70, Nr. 13, 31. März 1969, Columbus, Ohio, USA; HARDIE W. et al.: "Synthesis of 1,3-dioxolan-4 yl-alkyl guanidines", Seite 371, Spalte 1, Zusammenfassung Nr. 57 808u

CHEMICAL ABSTRACTS, Band 91, Nr. 5, 30. Juli 1979, Columbus, Ohio, USA; FAFF J. et al.: "Preliminary screening of anticholinergic effect of new 1,3-dioxolane and 1,3-dioxane derivatives", Seite 18, Spalte 1, Zusammenfassung Nr. 32 647m

CHEMIICAL ABSTRACTS, Band 101, Nr. 17, 22. October 1984, COlumbus, Ohio, USA; HSI R.S.P. et al.: "Carbon-14 and tritium labeled guanadrel sulfate", Seite 710, Spalte 2, Zusammenfassung Nr. 151 783n

## Beschreibung

Die Erfindung betrifft neue Aminomethylheterocyclen, mehrere Verfahren zu ihrer Herstellung sowie ihre Verwendung als Schädlingsbekämpfungsmittel.

Es ist bereits bekannt, daß bestimmte Aminomethyldioxolane, wie beispielsweise das 3-Isobutyl-2-methyl-3-(1-piperidinylmethyl)-dioxolan oder das 2-Methyl-2-nonyl-4-di-n-butylaminomethyldioxolan, fungizide Eigenschaften besitzen (vgl. EP 97 822).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue Aminomethylheterocyclen der allgemeinen Formel (I),

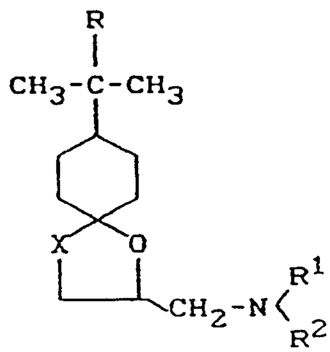

(I)

in welcher

- X für Sauerstoff oder Schwefel steht,
- R für Wasserstoff, für geradkettiges oder verzweigtes Alkyl mit 1 bis 6 Kohlenstoffatomen oder für jeweils gegebenenfalls ein- bis dreifach, gleich oder verschieden durch Alkyl mit 1 bis 4 Kohlenstoffatomen und/oder Halogen substituiertes Phenyl oder Cyclohexyl steht und
- $R^1$ und $R^2$ unabhängig voneinander jeweils für Wasserstoff; für jeweils geradkettiges oder verzweigtes Alkyl mit 1 bis 12 Kohlenstoffatomen, Alkenyl mit 1 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls in Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:
  Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen:
  Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder
- $R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen,

deren Säureadditionssalze, deren geometrische und optische Isomere und Isomerengemische.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Aminomethylheterocyclen der allgemeinen Formel (I),

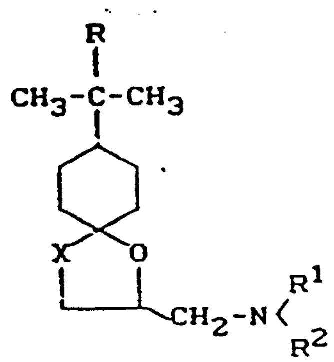

(I)

in welcher

X für Sauerstoff oder Schwefel steht,

R für Wasserstoff, für geradkettiges oder verzweigtes Alkyl mit 1 bis 6 Kohlenstoffatomen oder für jeweils gegebenenfalls ein- bis dreifach, gleich oder verschieden durch Alkyl mit 1 bis 4 Kohlenstoffatomen und/oder Halogen substituiertes Phenyl oder Cyclohexyl steht und

$R^1$ und $R^2$ unabhängig voneinander jeweils für Wasserstoff; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls in Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:
Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen:
Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen,

deren Säureadditionssalze, deren geometrische und optische Isomere und Isomerengemische.

erhält, wenn man

(a) substituierte Heterocyclen der Formel (II),

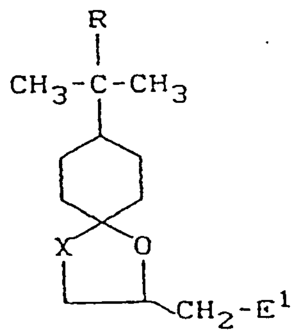

(II)

in welcher

R und X die oben angegebene Bedeutung haben und

$E^1$ für eine elektronenanziehende Abgangsgruppe steht,

mit Aminen der Formel (III),

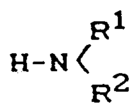

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels umsetzt, oder wenn man

(b) die nach Verfahren (a) erhältlichen Aminomethylheterocyclen der Formel (Ia),

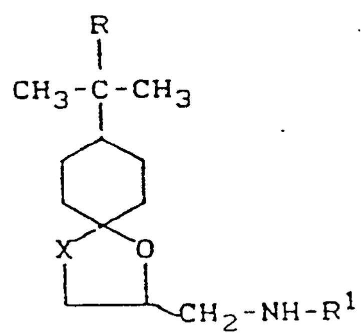

(Ia)

in welcher

R, $R^1$ und X die oben angegebene Bedeutung haben,

mit Alkylierungsmitteln der Formel (IV),

$R^{2-1}$-$E^2$ (IV)

in welcher

$R^{2-1}$ jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil, für jeweils gegebenenfalls im Cycloalkylteil einfach bis mehrfach, gleich oder verschieden substituiertes Cycloalkyl oder Cycloalkylalkyl mit jeweils

3 bis 7 Kohlenstoffatomen im Cycloalkylteil und gegebenenfalls 1 bis 4 Kohlenstoffatomen im geradkettigen oder verzweigten Alkylteil, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl- bzw. Alkenylteil, wobei als Arylsubstituenten jeweils infrage kommmen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen und

$E^2$ für eine elektronenanziehende Abgangsgruppe steht,

gegebenenfalls in Gegenwart eines Verdünnungsmittels und gegebenenfalls in Gegenwart eines Säurebindemittels umsetzt und gegebenenfalls anschließend eine Säure addiert oder eine physikalische Trennmethode anschließt.

Schließlich wurde gefunden, daß die neuen Aminomethylheterocyclen der allgemeinen Formel (I) eine Wirkung gegen Schädlinge, insbesondere gegen pilzliche Schädlinge besitzen.

Überraschenderweise zeigen die erfindungsgemäßen Aminomethylheterocyclen der allgemeinen Formel (I) eine bessere fungizide Wirksamkeit als die aus dem Stand der Technik bekannten Aminomethyldioxolane, wie beispielsweise das 2-Isobutyl-2-methyl-4-(1-piperidinylmethyl)-1,3-dioxolan oder das 2-Methyl-2-nonyl-4-di-n-butylaminomethyl-1,3-dioxolan, welches chemisch und wirkungsmäßig naheliegende Verbindungen sind.

Die erfindungsgemäßen Aminomethylheterocyclen sind durch die Formel (I) allgemein definiert. Bevorzugt sind Verbindungen der Formel (I), bei welchen

X für Sauerstoff oder Schwefel steht,

R für Wasserstoff, Methyl, Ethyl, Neopentyl, Cyclohexyl oder Phenyl steht und

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl,, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylethyl, Ethoxycarbonylpropyl, Propoxycarbonylmethyl, Propoxycarbonylehtyl, Propoxycarbonylpropyl, Dioxolanylmethyl, Dioxolanylethyl, Dioxanylmethyl, Dioxanylethyl, Oxolanylmethyl, Oxolanylethyl, für jeweils gegebenenfalls ein- bis fünffach, gleich oder verschieden durch Fluor, Chlor, Brom, Methyl, Ethyl, n- oder i-Propyl, n-, i-, s-und/oder t-Butyl substituiertes Cyclopropyl, Cyclopropylmethyl,, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl steht, wobei als Substituenten jeweils infrage kommen: Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

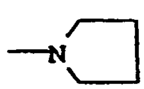 ; 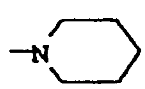 ; 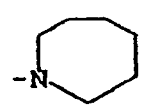 ; 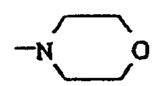

oder 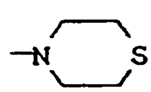

stehen, wobei als Substituenten infrage kommen: Methyl, Ethyl oder Hydroxymethyl.

Ganz besonders bevorzugt sind Verbindungen der Formel (I), bei welchen

X für Sauerstoff oder Schwefel steht,

R für Wasserstoff, Methyl oder Ethyl steht und

$R^1$ und $R^2$ unabhängig voneinander jeweils für Wasserstoff, Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Oxolanylmethyl, Oxolanylethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl steht oder

$R^1$ und $R^2$ gemeinsam mit dem Stickstoffatom, an welches sie gebunden sind, für einen gegebenenfalls einbis dreifach, gleich oder verschieden substituierten Heterocyclus der Formel

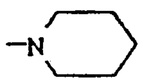 ; 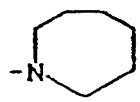 ; 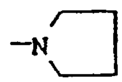 oder 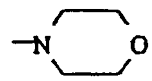

stehen,

wobei als Substituenten infrage kommen: Methyl, Ethyl, Hydroxymethyl.

Halogen bedeutet auch in den Zusammensetzungen Fluor, Chlor, Brom und Iod, insbesondere Fluor, Chlor oder Brom, wenn nicht anders definiert.

Bevorzugte erfindungsgemäße Verbindungen sind auch Additionsprodukte aus Säuren und denjenigen Aminomethylheterocyclen der Formel (I), in denen die Substituenten X, R, $R^1$ und $R^2$ die Bedeutungen haben, die bereits für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, und Sulfonsäuren, wie p-Toluolsulfonsäure und 1,5-Naphthalindisulfonsäure und ferner Saccharin.

Im einzelnen seien außer den bei den Herstellungsbeispielen genannten Verbindungen die folgenden Aminomethylheterocyclen der allgemeinen Formel (I) genannt:

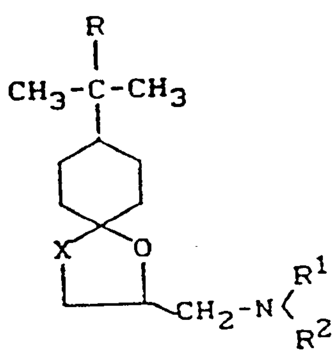

(I)

| R | X | 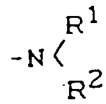 |
|---|---|---|
| $CH_3$ | S | 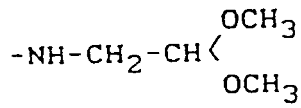 |
| $CH_3$ | S | 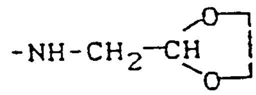 |
| $CH_3$ | S | 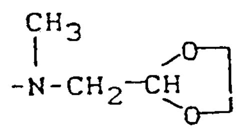 |

| R | X | 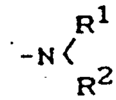 |
|---|---|---|
| $CH_3$ | S | 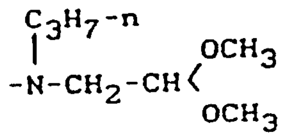 |
| $CH_3$ | S | 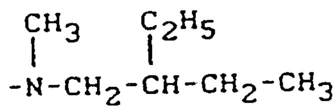 |
| $CH_3$ | S | 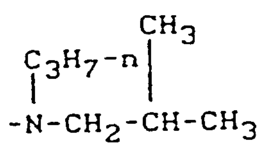 |
| $CH_3$ | S | 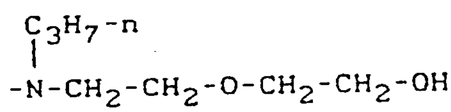 |
| $CH_3$ | S | 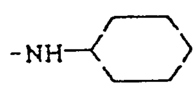 |
| $CH_3$ | S | 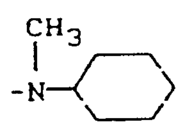 |
| $CH_3$ | S | 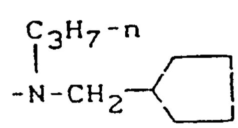 |

| R | X | 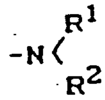 |
| --- | --- | --- |
| $CH_3$ | S | 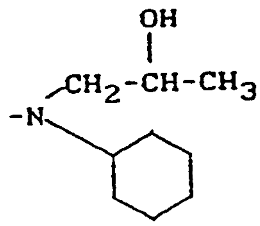 |
| $CH_3$ | S | 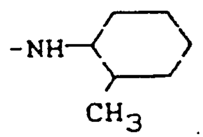 |
| $CH_3$ | S | 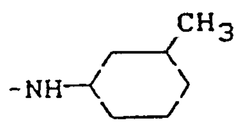 |
| $C_2H_5$ | S | 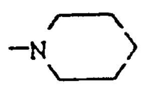 |
| $C_2H_5$ | S | 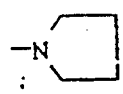 |
| $C_2H_5$ | S | 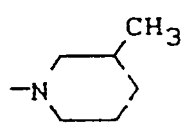 |
| $C_2H_5$ | S | 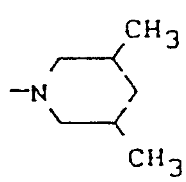 |

| R | X | 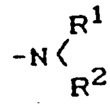 |
|---|---|---|
| $C_2H_5$ | S | 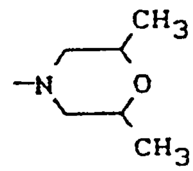 |
| $C_2H_5$ | S | 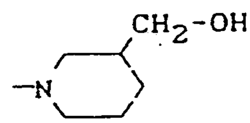 |
| $C_2H_5$ | S | 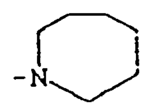 |
| $C_2H_5$ | S | 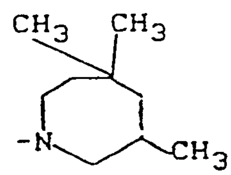 |
| $C_2H_5$ | S | $-NH-CH_2-C{\equiv}CH$ |
| $C_2H_5$ | S | $-NH-CH_2-CH_2-C{\equiv}CH$ |
| $C_2H_5$ | S | 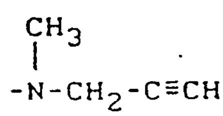 |

| R | X | 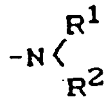 |
|---|---|---|
| $CH_3$ | O | 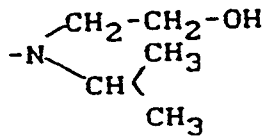 |
| $CH_3$ | O | 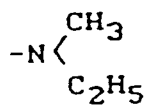 |
| $CH_3$ | O | 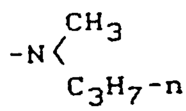 |
| $CH_3$ | O | 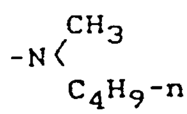 |
| $CH_3$ | O | 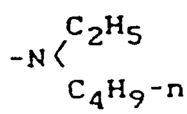 |
| $CH_3$ | O | 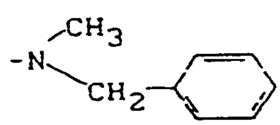 |
| $CH_3$ | O | 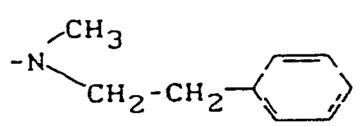 |

| R | X | $-N\begin{smallmatrix}R^1\\R^2\end{smallmatrix}$ |
|---|---|---|
| $CH_3$ | O | 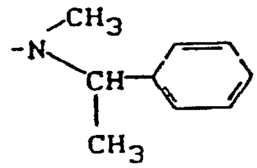 |
| $CH_3$ | O | 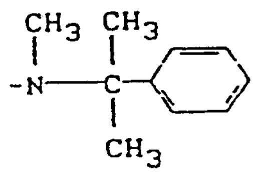 |
| $CH_3$ | O | 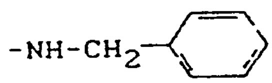 |
| $CH_3$ | O | 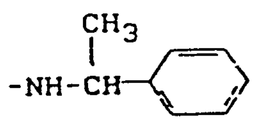 |
| $CH_3$ | O | 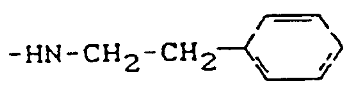 |
| $CH_3$ | O | 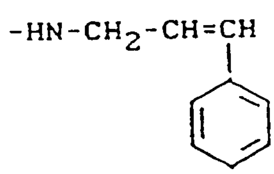 |
| $CH_3$ | O | 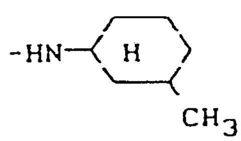 |

| R | X | 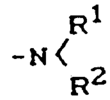 |
|---|---|---|
| $CH_3$ | O | 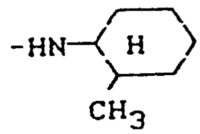 |
| $CH_3$ | O | 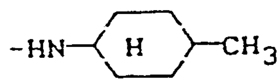 |
| 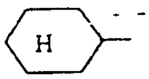 | O | 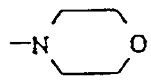 |
| 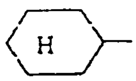 | O | 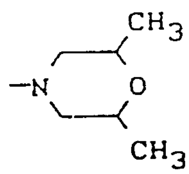 |
| 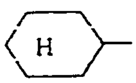 | O | 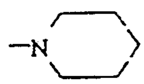 |
| 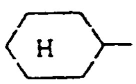 | O | 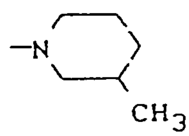 |
| 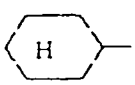 | O | 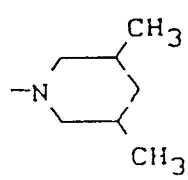 |

| R | X | $-N<^{R^1}_{R^2}$ |
|---|---|---|
| 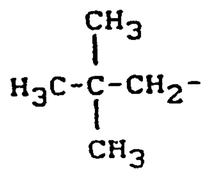 | O | 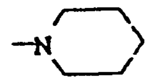 |
| $C_2H_5$ | O | 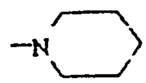 |
| $C_2H_5$ | O | 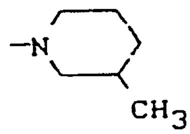 |
| 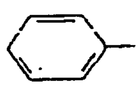 | O | 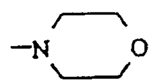 |

Verwendet man beispielsweise 8-t-Butyl-2-chlormethyl-1,4-dioxaspiro[4,5]decan und Piperidin als Ausgangsstoffe, so laßt sich der Reaktionsablauf des erfindungsgemäßen Verfahrens (a) durch das folgende Formelschema darstellen:

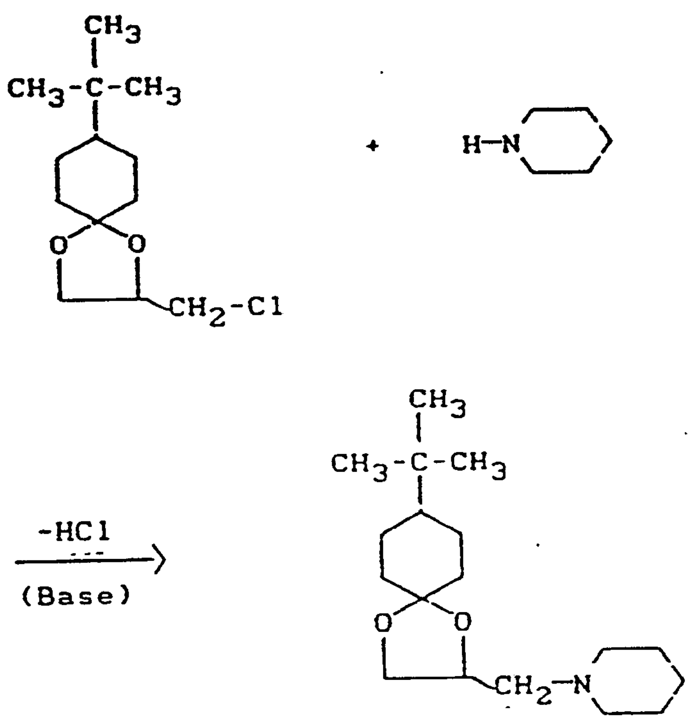


Verwendet man beispielsweise 8-t-Butyl-2-methylaminomethyl-1,4-dioxaspiro[4,5]decan und Allylbromid

als Ausgangsstoffe, so läßt sich der Reaktionsablauf des erfindungsgemäßen Verfahrens (b) durch das folgende Formelschema darstellen:

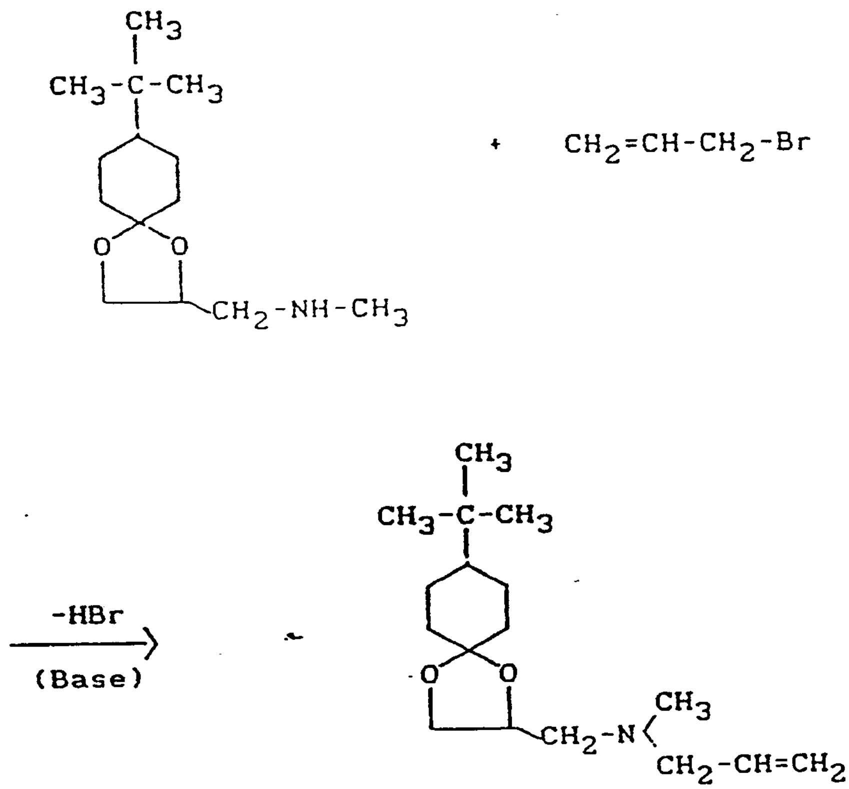


Die zur Durchführung des erfindungsgemäßen Verfahrens (a) als Ausgangsstoffe benötigten substituierten Heterocyclen sind durch die Formel (II) allgemein definiert. In dieser Formel (II) stehen R und X vorzugsweise für diejenigen Reste, die bereits im Zusammenhang mit der Beschreibung der erfindungsgemäßen Stoffe der Formel (I) für diese Substituenten genannt wurden.

$E^1$ steht vorzugsweise für Halogen, insbesondere für Iod, Chlor oder Brom oder für gegebenenfalls durch Halogen, wie Fluor, Chlor, Brom oder Iod, substituiertes Alkylsulfonyloxy oder für gegebenenfalls u.a. durch Alkyl mit 1 bis 4 Kohlenstoffatomen substituiertes Arylsulfonyloxy, wie beispielsweise Methansulfonyloxy, Trifluormethansulfonyloxy oder p-Toluolsulfonyloxy,

Die substituierten Heterocyclen der Formel (II) sind bekannt (vgl. z.B. J. org. Chem. 38, 834-835 [1973]) oder lassen sich in Analogie zu bekannten Verfahren herstellen (vgl z.B. Tetrahedron Lett. 23, 47-50, [1982]: Liebigs Ann. Chem. 1984, 1298-1301; Z. Naturforsch. B, Anorg. Chem., Org. Chem. 4013, 393-397 [1985] oder J. org. Chem. 51, 1894-1897 [1986] sowie die Herstellungsbeispiele), beispielsweise wenn man allgemein bekannte cyclische Ketone der Formel (V),

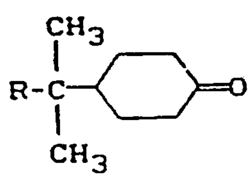
(V)

in welcher

R die oben angegebene Bedeutung hat,

mit allgemein bekannten Alkoholen der Formel (VI),

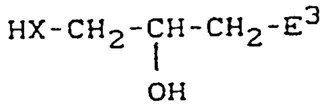

(VI)

in welcher

X die oben angegebene Bedeutung hat und

$E^3$ für Halogen oder Hydroxy steht,

gegebenenfalls in Gegenwart eines Verdünnungsmittels, wie beispielsweise Toluol, und gegebenenfalls in Gegenwart eines sauren Katalysators, wie beispielsweise p-Toluolsulfonsäure, bei Temperaturen zwischen 40 °C und 150 °C cyclisiert und gegebenenfalls in den Fällen, wo $E^3$ in Formel (VI) für eine Hydroxygruppe steht, in einer 2. Stufe die so erhältlichen Hydroxymethylheterocyclen der Formel (VII),

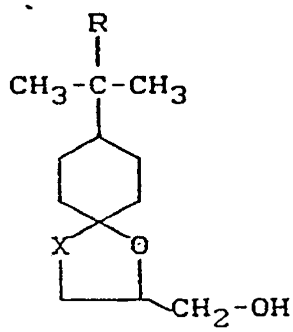

(VII)

in welcher

X und R die oben angegebene Bedeutung haben,

mit gegebenenfalls substituierten Alkyl- oder Arylsulfonylhalogeniden der Formel (VIII),

$Z\text{-}SO_2\text{-}Hal$ (VIII)

in welcher

Hal für Halogen, insbesondere für Chlor steht und

Z für gegebenenfalls durch Halogen, wie Fluor, Chlor, Brom oder Iod substituiertes Alkyl oder für gegebenenfalls durch Alkyl mit 1 bis 4 Kohlenstoffatomen substituiertes Aryl, wie insbesondere Methyl, Trifluormethyl oder 4-Methylphenyl steht,

gegebenenfalls in Gegenwart eines Verdünnungsmittels, wie beispielsweise Diethylether, und gegebenenfalls in Gegenwart eines Säurebindemittels, wie beispielsweise Pyridin oder Triethylamin, bei Temperaturen zwischen - 20 °C und + 100 °C umsetzt.

Die dabei erhältlichen geometrischen Isomeren lassen sich entweder als Gemische im erfindungsgemäßen Verfahren (a) weiter umsetzen oder mit üblichen Trennmethoden (Chromatographie, Kristallisation) auftrennen.

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) für diese Substituenten genannt wurden.

Die Amine der Formel (III) sind allgemein bekannte Verbindungen der organischen Chemie.

Die zur Durchführung des erfindungsgemäßen Verfahrens (b) als Ausgangsstoffe benötigten Aminomethylheterocyclen sind durch die Formel (Ia) allgemein definiert. In dieser Formel (Ia) stehen X, R und $R^1$ vorzugsweise für diejenigen Reste, die bereits im Zusammenhang mit der Beschreibung der erfindungsgemäßen Stoffe der Formel (I) für diese Substituenten genannt wurden.

Die Aminomethylheterocyclen der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{2-1}$ bevorzugt für Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl,

Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Dioxanylethyl, Oxolanylmethyl, Oxolanylethyl, für gegebenenfalls jeweils ein- bis fünffach, gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Benzyl oder Phenylethyl, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl.

$R^{2-1}$ steht ganz besonders bevorzugt für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Cyclopropylmethyl, Oxolanylmethyl, Oxolanylethyl, Dichlorcyclopropylmethyl, Dimethylcyclopropylmethyl, Dichlordimethylcyclopropylmethyl, Cyclopentyl, Cyclohexyl oder Cyclohexylmethyl,

$E^2$ steht vorzugsweise für Halogen, insbesondere für Chlor, Brom oder Iod oder für jeweils gegebenenfalls durch Halogen, wie Fluor, Chlor, Brom oder Iod substituiertes Alkylsulfonyloxy, Alkoxysulfonyloxy mit jeweils 1 bis 4 Kohlenstoffatomen oder für gegebenenfalls durch z.B. Alkyl mit 1 bis 4 Kohlenstoffatomen substituiertes Arylsulfonyloxy, wie beispielsweise Methansulfonyloxy, Methoxysulfonyloxy oder p-Toluolsulfonyloxy.

Die Alkylierungsmittel der Formel (IV) sind ebenfalls allgemein bekannte Verbindungen der organischen Chemie oder erhältlich in Analogie zu allgemein bekannten Verfahren.

Als Verdünnungsmittel zur Durchführung der erfindungsgemäßen Verfahren (a) und (b) kommen inerte organische Lösungsmittel oder wäßrige Systeme infrage.
Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide, wie Dimethylsulfoxid oder Alkohole, wie Methanol, Ethanol oder Propanol.

Die erfindungsgemäßen Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid oder Trimethylbenzylammoniumchlorid. Es ist auch möglich die erfindungsgemäßen Verfahren (a) und (b) ohne Zusatz eines Lösungsmittels durchzuführen.

Als Säurebindemittel zur Durchführung der erfindungsgemäßen Verfahren (a) und (b) kommen alle üblicherweise verwendbaren anorganischen und organischen Basen infrage. Vorzugsweise verwendet man Alkalimetallhydroxide, -alkoholate, -carbonate oder -hydrogencarbonate, wie beispielsweise Natriumhydroxid, Natriummethylat, Natriumethylat, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist auch möglich, die als Reaktionsteilnehmer verwendeten Amine der Formeln (III) bzw. (Ia) in entsprechendem Überschuß gleichzeitig als Säurebindemittel einzusetzen.

Die Reaktionstemperaturen können bei der Durchführung der erfindungsgemäßen Verfahren (a) und (b) in einem größeren Bereich variiert werden. Im allgemeinen arbeitet man bei Temperaturen zwischen + 20 °C und + 200 °C, vorzugsweise bei Temperaturen zwischen 80 °C und + 180 °C.

Die erfindungsgemäßen Verfahren (a) und (b) werden im allgemeinen bei Normaldruck durchgeführt. Es

ist jedoch auch möglich unter erhöhtem Druck im Bereich zwischen 1 und 10 atm zu arbeiten. Die Arbeitsweise unter erhöhtem Druck empfiehlt sich insbesondere, wenn ein oder mehrere Reaktionsteilnehmer bei Normaldruck und der erforderlichen Reaktionstemperatur gasförmig vorliegen.

Zur Durchführung des erfindungsgemäßen Verfahrens (a) setzt man pro Mol an substituiertem Heterocyclus der Formel (II) im allgemeinen 1,0 bis 10,0 Mol, vorzugsweise 1,0 bis 5,0 Mol an Amin der Formel (III) und gegebenenfalls 1,0 bis 10,0 Mol, vorzugsweise 1,0 bis 5,0 Mol an Säurebindemittel, und gegebenenfalls 0,1 bis 1,0 Mol an Phasentransferkatalysator ein.

Zu Durchführung des erfindungsgemäßen Verfahrens (b) setzt man pro Mol an Aminomethylheterocyclus der Formel (Ia) im allgemeinen 1,0 bis 5,0 Mol, vorzugsweise 1,0, bis 2,0 Mol an Alkylierungsmittel der Formel (IV) und 1,0 bis 5,0 Mol, vorzugsweise 1,0 bis 2,0 Mol an Säurebindemittel, und gegebenenfalls 0,1 bis 1,0 Mol an Phasentransferkatalysator ein.

Die Reaktionsdurchführung, Aufarbeitung und Isolierung der Reaktionsprodukte der Formel (I) erfolgt in beiden Fällen nach üblichen Methoden.

Zur Herstellung von Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, und Sulfonsäuren, wie z.B. p-Toluolsulfonsäure und 1,5-Naphthalindisulfonsäure und außerdem Saccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch u.a. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Blattfleckenkrankheit der Gerste (Pyrenophora teres) oder gegen den Erreger der Blattfleckenkrankheit des Weizens (Cochliobolus sativus) sowie gegen Mehltau und Rostarten oder zur Bekämpfung von Krankheiten im Obst- und Gemüseanbau, wie beispielsweise gegen den Erreger des Apfelschorfes (Venturia inaequalis) einsetzen. Darüberhinaus zeigen die erfindungsgemäßen Wirkstoffe eine gute in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

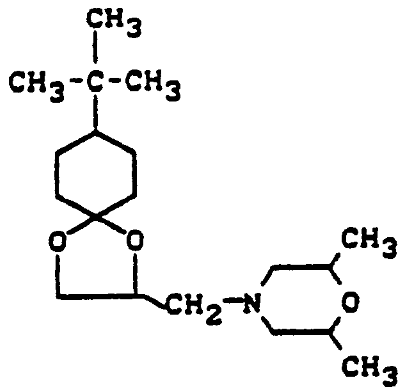

(Verfahren a)

12,3 g (0,05 Mol) 8-t-Butyl-2-chlormethyl-1,4-dioxaspiro[4,5]decan (cis-trans-Gemisch) und 23 g (0,2 Mol) cis-2,6-Dimethylmorpholin werden zusammen 15 Stunden bei 130 °C gerührt. Zur Aufarbeitung gibt man 100 ml Essigester zur erkalteten Reaktionsmischung, wäscht fünfmal mit jeweils 50 ml Wasser, trocknet über Natriumsulfat und entfernt das Lösungsmittel im Vakuum.

Man erhält 15,8 g (97 % der Theorie) an 8-t-Butyl-2-(2,6-dimethylmorpholin-4-yl-methyl)-1,4-dioxaspiro-[4,5] decan als Öl vom Brechungsindex $n_D^{20}$ : 1,4756, welches laut gaschromatographischer Analyse überwiegend als cis/cis-und cis/trans-Diastereomerengemisch vorliegt.

Herstellung der Ausgangsverbindung

Beispiel II-1:

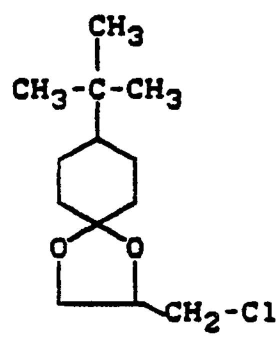

100g (0,648 Mol) 4-t-Butylcyclohexanon, 143,2 g (1,296 Mol) 3-Chlor-1,2-propandiol und 12,3 g (0,0648 Mol) p-Toluolsulfonsäure werden in 1 l Toluol 15 Stunden über einem Wasserabscheider unter Rückfluß erhitzt. Das erkaltete Reaktionsgemisch wird fünfmal mit jeweils 300 ml gesättigter wässriger Natriumhydrogencarbonatlösung gewaschen, über Natriumsulfat getrocknet und im Vakuum vom Lösungsmittel befreit.

Man erhält 159,5 g (99 % der Theorie) an 8-t-Butyl-2-chlormethyl-1,4-dioxaspiro[4,5]decan vom Brechungsindex $n_D^{20}$ : 1.4774, welches laut gaschromatographischer Analyse und Protonenkernresonanzspektrum als cis-trans-(55:45)-Gemisch vorliegt.

In entsprechender Weise und gemäß den allgemeinen Angaben zur Herstellung erhält man die folgenden Aminomethylheterocyclen der allgemeinen Formel (I):

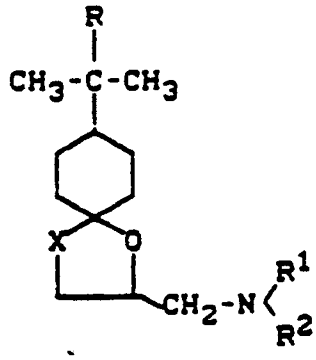

(I)

| Bsp. Nr. | X | R | 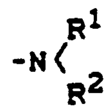 | physikalische Eigenschaften |
|---|---|---|---|---|
| 2 | O | $CH_3$ | 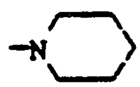 | $n_D^{20}$: 1,4830 |
| 3 | O | $CH_3$ | 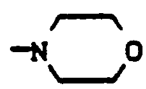 | $n_D^{20}$: 1,4833 |
| 4 | O | $CH_3$ | 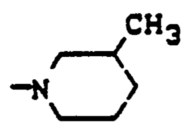 | $n_D^{20}$: 1,4801 |
| 5 | O | $CH_3$ | 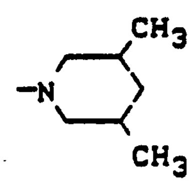 | $n_D^{20}$: 1,4767 |

| Bsp. Nr. | X | R | $-N\langle^{R^1}_{R^2}$ | physikalische Eigenschaften |
|---|---|---|---|---|
| 6 | O | $CH_3$ | 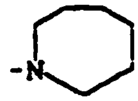 | $n_D^{20}$:1,4867 |
| 7 | O | $CH_3$ | 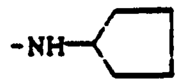 | $n_D^{20}$:1,4856 |
| 8 | O | $CH_3$ | 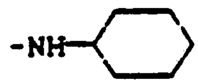 | $n_D^{20}$:1,4861 |
| 9 | S | $CH_3$ | $-NH-CH_2-CH(CH_3)_2$ | $n_D^{20}$:1,4916 |
| 10 | S | $CH_3$ | $-NH-CH_2-CH(CH_3)_2$ | $n_D^{20}$:1,5298 |
| 11 | S | $CH_3$ | 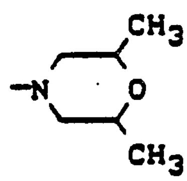 | $n_D^{20}$:1,5012 |
| 12 | S | $CH_3$ | 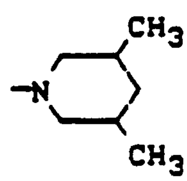 | Fp. 43°C |

| Bsp. Nr. | X | R | $-N<^{R^1}_{R^2}$ | physikalische Eigenschaften |
|---|---|---|---|---|
| 13 | S | $CH_3$ | 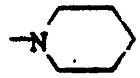 | Fp. 48° C |
| 14 | S | $CH_3$ | $-NH-(CH_2)_3-OC_2H_5$ | $n_D^{20}$:1,4959 |
| 15 | O | $CH_3$ | 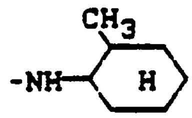 | $n_D^{20}$: 1,4872 |
| 16 | O | $CH_3$ | 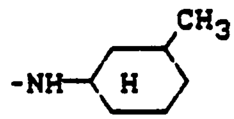 | $n_D^{20}$: 1,4872 |
| 17 | O | $CH_3$ | 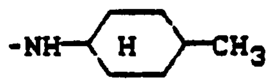 | $n_D^{20}$: 1,4808 |
| 18 | O | $C_2H_5$ | 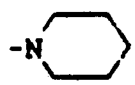 | $n_D^{20}$: 1,4851 |
| 19 | O | $C_2H_5$ | 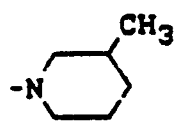 | $n_D^{20}$: 1,4814 |
| 20 | O | $C_2H_5$ | 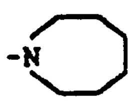 | $n_D^{20}$: 1,4886 |
| 21 | O | $C_2H_5$ | 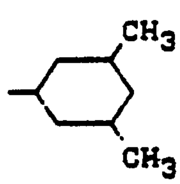 | $n_D^{20}$: 1,4793 |

| Bsp. Nr. | X | R | $-N{<}{R^1 \atop R^2}$ | physikalische Eigenschaften |
|---|---|---|---|---|
| 22 | O | $C_2H_5$ | 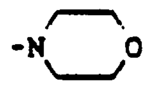 | $n_D^{20}$: 1,4847 |
| 23 | O | $C_2H_5$ | 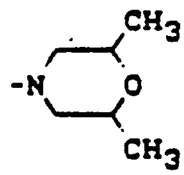 | $n_D^{20}$: 1,4785 |
| 24 | O | $CH_3$ | 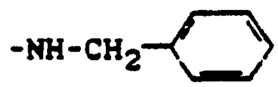 | $n_D^{20}$: 1,5088 |
| 25 | O | $CH_3$ | 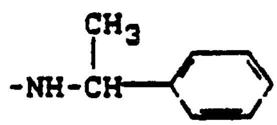 | $n_D^{20}$: 1,5088 |
| 26 | O | $CH_3$ | 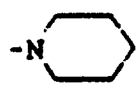 | $n_D^{20}$: 1,4858 |
| 27 | O | $CH_3$ | 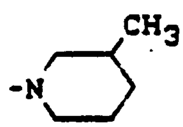 | $n_D^{20}$: 1,4874 |
| 28 | O | $CH_3$ | 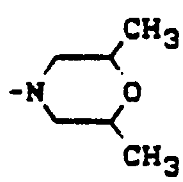 | $n_D^{20}$: 1,4829 |
| 29 | O | 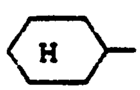 | 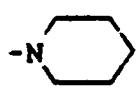 | Fp. 37-48° C |

| Bsp. Nr. | X | R | 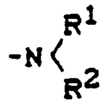 | physikalische Eigenschaften |
|---|---|---|---|---|
| 30 | O | 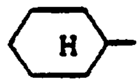 | 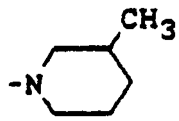 | $n_D^{20}$: 1,4993 |
| 31 | O | 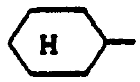 | 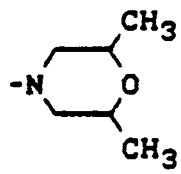 | $n_D^{20}$: 1,4958 |
| 32 | O | $CH_3$ | 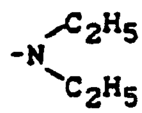 | $n_D^{20}$: 1,4660 |
| 33 | O | $CH_3$ | $-NH-(CH_2)_3-OC_2H_5$ | $n_D^{20}$: 1,4700 |
| 34 | O | $CH_3$ | $-NH-CH_2-CH(C_2H_5)_2$ | $n_D^{20}$: 1,4672 |
| 35 | O | $CH_3$ | 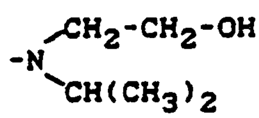 | $n_D^{20}$: 1,4757 |
| 36 | O | $CH_3$ | 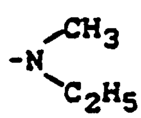 | $n_D^{20}$: 1,4662 |
| 37 | O | $CH_3$ | 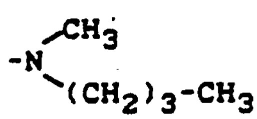 | $n_D^{20}$: 1,4653 |
| 38 | O | $CH_3$ | 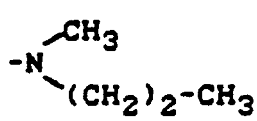 | $n_D^{20}$: 1,4663 |

| Bsp. Nr. | X | R | 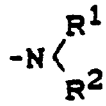 | physikalische Eigenschaften |
|---|---|---|---|---|
| 39 | O | $CH_3$ | 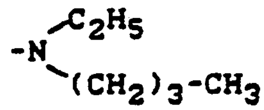 | $n_D^{20}$: 1,4649 |
| 40 | O | $CH_3$ | 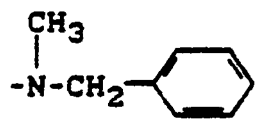 | $n_D^{20}$: 1,5079 |
| 41 | O | 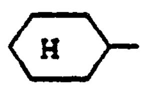 | 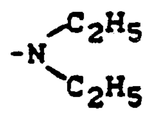 | $n_D^{20}$: 1,4893 |
| 42 | O | $CH_3$ | $-NH-(CH_2)_2-OCH_3$ | $n_D^{20}$: 1,4692 |
| 43 | O | $CH_3$ | $-NH-CH_3$ | Kp. 116-120° C/ 0,9 mm |
| 44 | O | $CH_3$ | 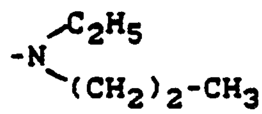 | $n_D^{20}$: 1,4662 |
| 45 | O | $(CH_3)_3C-CH_2-$ | 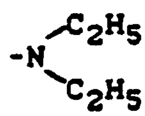 | $n_D^{20}$: 1,4733 |
| 46 | O | $C_2H_5$ | 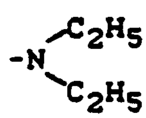 | $n_D^{20}$: 1,4651 |
| 47 | O | $CH_3$ | 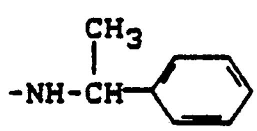 | $n_D^{20}$: 1,5050 |

| Bsp. Nr. | X | R | 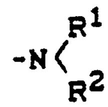 | physikalische Eigenschaften |
|---|---|---|---|---|
| 48 | O | $CH_3$ | 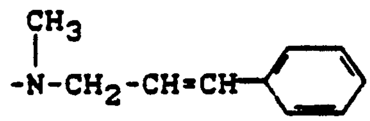 | $n_D^{20}$: 1,5254 |
| 49 | O | $CH_3$ | 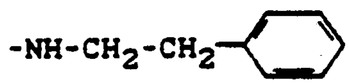 | $n_D^{20}$: 1,5123 |
| 50 | O | $CH_3$ | 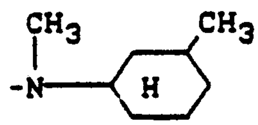 | |
| 51 | O | $CH_3$ | 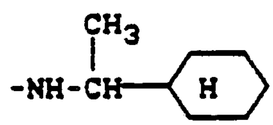 | $n_D^{20}$: 1,4836 |
| 52 | O | $C_2H_5$ | 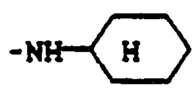 | $n_D^{20}$: 1,4871 |
| 53 | O | $C_2H_5$ | 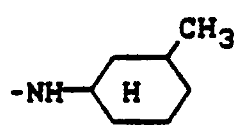 | $n_D^{20}$: 1,4838 |
| 54 | O | $CH_3$ | 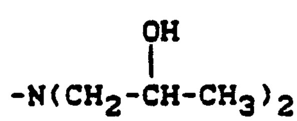 | $n_D^{20}$: 1,4788 |
| 55 | O | $C_2H_5$ | $-NH-(CH_2)_2-OCH_3$ | $n_D^{20}$: 1,4720 |

| Bsp. Nr. | X | R | 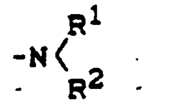 | physikalische Eigenschaften |
|---|---|---|---|---|
| 56 | O | $C_2H_5$ | $-NH-(CH_2)_3-CH_3$ | $n_D^{20}$: 1,4708 |
| 57 | O | $CH_3$ | 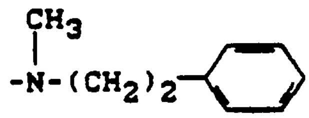 | Kp. 157° C |
| 58 | O | $CH_3$ | 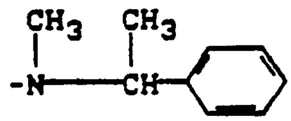 | |
| 59 | O | $CH_3$ | 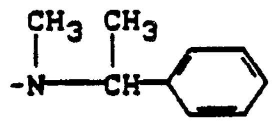 | |
| 60 | O | $CH_3$ | 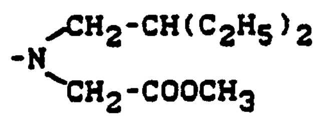 | $n_D^{20}$: 1,4706 |
| 61 | O | $CH_3$ | 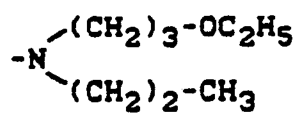 | $n_D^{20}$: 1,4647 |
| 62 | O | $CH_3$ | 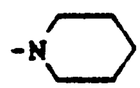 x 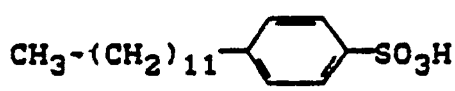 | |

| Bsp. Nr. | X | R | $-N<^{R^1}_{R^2}$ | physikalische Eigenschaften |
|---|---|---|---|---|
| 63 | O | $CH_3$ | 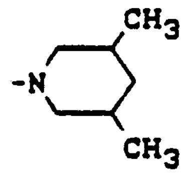 x 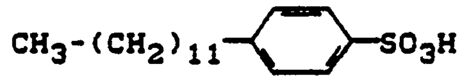 | |
| 64 | O | $CH_3$ | 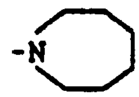 x 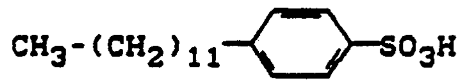 | |
| 65 | O | $CH_3$ | 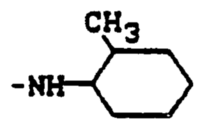 x 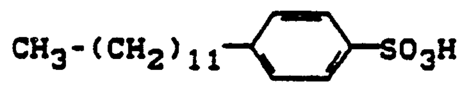 | |
| 66 | O | $CH_3$ | 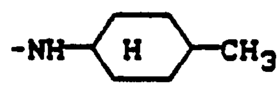 x 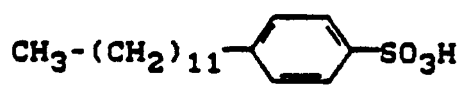 | |

| Bsp. Nr. | X | R | $-N\langle^{R^1}_{R^2}$ | physikalische Eigenschaften |
|---|---|---|---|---|
| 67 | O | $CH_3$ | 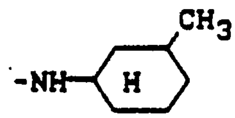<br>x $CH_3-(CH_2)_{11}-$ 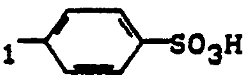 | |
| 68 | O | $CH_3$ | 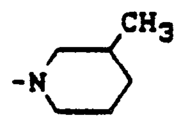<br>x $CH_3-(CH_2)_{11}-$ 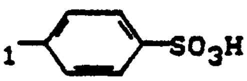 | |
| 69 | O | $CH_3$ | 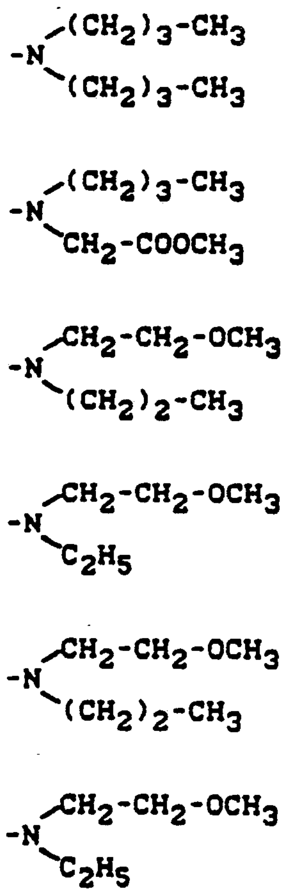 | Kp. 136° C |
| 70 | O | $C_2H_5$ | | $n_D^{20}$: 1,4713 |
| 71 | O | $CH_3$ | | $n_D^{20}$: 1,4651 |
| 72 | O | $C_2H_5$ | | $n_D^{20}$: 1,4685 |
| 73 | O | $C_2H_5$ | | $n_D^{20}$: 1,4687 |
| 74 | O | $CH_3$ | | $n_D^{20}$: 1,4659 |

| Bsp. Nr. | X | R | 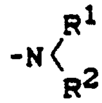 | physikalische Eigenschaften |
|---|---|---|---|---|
| 75 | S | $CH_3$ | 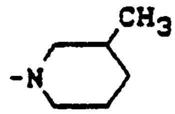 | $n_D^{20}$: 1,4988 |
| 76 | S | $CH_3$ | 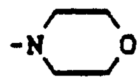 | Fp. 37-57° C |
| 77 | S | $CH_3$ | 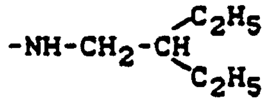 | $n_D^{20}$: 1,4016 |
| 78 | S | $CH_3$ | 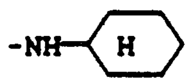 | $^1H$-NMR*): 4,2-4,4<br>3,0-3,1<br>2,7-2,9 |
| 79 | S | $CH_3$ | 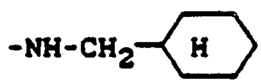 | $n_D^{20}$: 1,5058 |
| 80 | S | $CH_3$ | 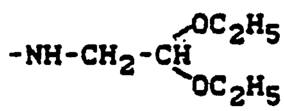 | $n_D^{20}$: 1,4870 |
| 81 | S | $CH_3$ | 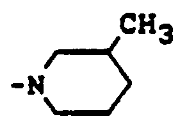<br>x 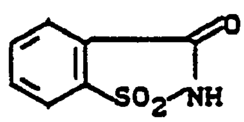 | $^1H$-NMR*): 4,6-4,9 (m)<br>3,5-4,1 (m) |
| 82 | S | $CH_3$ | 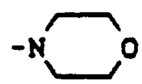<br>x 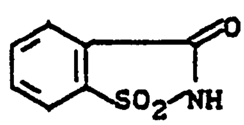 | $^1H$-NMR*): 4,6-4,9 (m)<br>4,05-4,1 (m)<br>3,05-3,8 (m) |

| Bsp. Nr. | X | R | 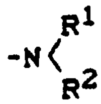 | physikalische Eigenschaften |
|---|---|---|---|---|
| 83 | S | $CH_3$ | 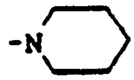 x 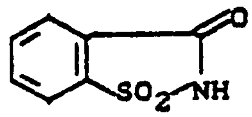 | $^{1}$H-NMR*): 4,6-4,9 (m) 3,6-4,05 (m) |
| 84 | S | $CH_3$ | 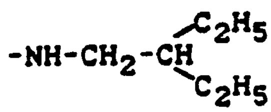 | $^{1}$H-NMR*): 4,6-4,8 (m) 3,5-3,7 (m) 3,1-3,3 (m) 2,7-2,9 (m) |
| 85 | S | $CH_3$ | 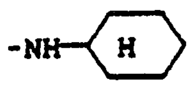 x 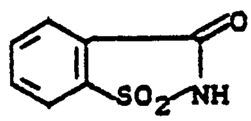 | $^{1}$H-NMR*): 4,6-4,8 (m) 3,1-3,6 (m) 2,7-2,9 (m) |
| 86 | S | $CH_3$ | 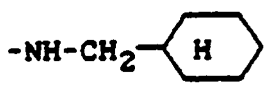 x 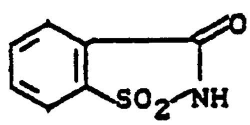 | $^{1}$H-NMR*): 4,6-4,8 (m) 3,5-3,7 (m) 3,0-3,3 (m) 2,7-2,9 (m) |
| 87 | S | $CH_3$ | 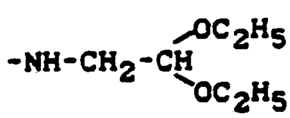 x 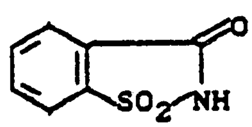 | $^{1}$H-MNR*): 4,95-5,05 (m) 4,6-4,8 (m) 3,5-3,9 (m) 3,1-3,5 (m) |
| 88 | S | $C_2H_5$ | 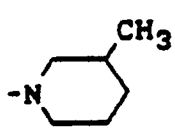 | $n_D^{20}$: 1,5058 |

| Bsp. Nr. | X | R | $-N\langle^{R^1}_{R^2}$ | physikalische Eigenschaften |
|---|---|---|---|---|
| 89 | S | $C_2H_5$ | 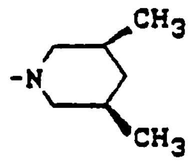 | $n_D^{20}$: 1,5027 |
| 90 | S | $C_2H_5$ | 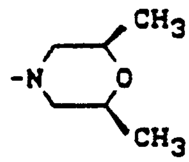 | $n_D^{20}$: 1,5027 |
| 91 | S | $C_2H_5$ | 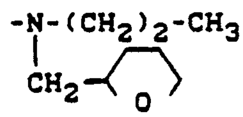 | $n_D^{20}$: 1,5027 |
| 92 | S | $C_2H_5$ | 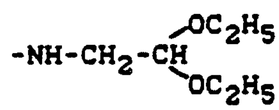 | $n_D^{20}$:1.4906 |
| 93 | S | $C_2H_5$ | $-NH-(CH_2)_3-OC_2H_5$ | $n_D^{20}$: 1,4969 |
| 94 | S | $C_2H_5$ | 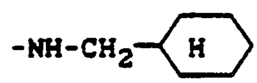 | $n_D^{20}$: 1,5099 |
| 95 | S | $C_2H_5$ | 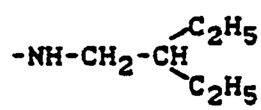 | $n_D^{20}$: 1,4958 |
| 96 | S | $C_2H_5$ | 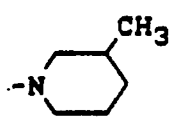 x 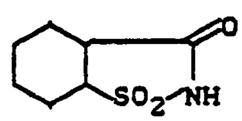 | $^1$H-NMR*): 4,6-4,9(m) 3,5-4,0(m) |
| 97 | S | $C_2H_5$ | 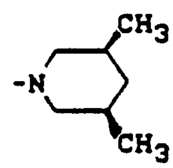 x 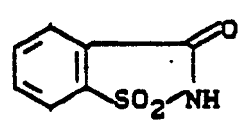 | $^1$H-NMR*): 4,6-4,9 3,5-3,9 |

| Bsp. Nr. | X | R | $-N<^{R^1}_{R^2}$ | physikalische Eigenschaften |
|---|---|---|---|---|
| 98 | S | $C_2H_5$ | 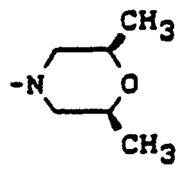 | $^1$H-NMR*): 4,6-4,9 (m); 4,1-4,3 (m); 3,5-3,9 (m); 3,0-3,25 (m). |
| 99 | S | $C_2H_5$ | 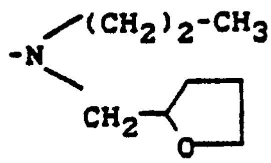 | $^1$H-NMR*): 4,6-4,9 (m) 4,3-4,5 (m) 3,1-3,9 (m) |
| | | | x 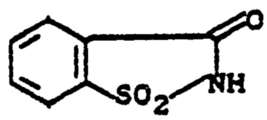 | |
| 100 | S | $C_2H_5$ | 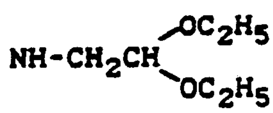 | $^1$H-NMR*): 3,15-3,9 (m); 4,6-4,8 (m); 4,95-5,05 (m) |
| | | | x 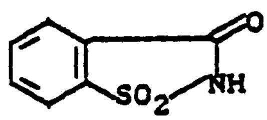 | |
| 101 | S | $C_2H_5$ | $-NH-(CH_2)_3-O-C_2H_5$ | $^1$H-NMR*): 4,6-4,8 (m); 3,1-3,7 (m). |
| | | | x 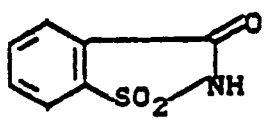 | |
| 102 | S | $C_2H_5$ | 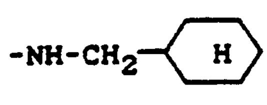 | $^1$H-NMR*): 4,5-4,8 (m); 3,5-3,75 (m); 3,0-3,25 (m); 2,7-2,9 (m); |
| | | | x 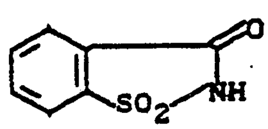 | |

| Bsp. Nr. | X | R | 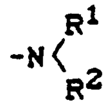 | physikalische Eigenschaften |
|---|---|---|---|---|
| 103 | S | $C_2H_5$ | 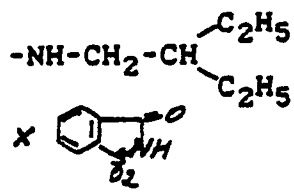 | $^1$H-NMR*): 4,6-4,8 (m); 3,5-3,75 (m); 3,1-3,3 (m). |
| 104 | O | $C_2H_5$ | 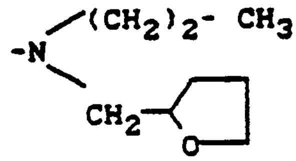 | $n_D^{20}$: 1,4802 |
| 105 | O | H | 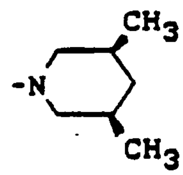 | $n_D^{20}$: 1,4759 |
| 106 | O | H | 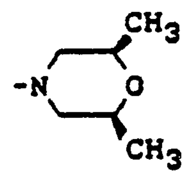 | $n_D^{20}$: 1,4750 |
| 107 | O | $C_2H_5$ | 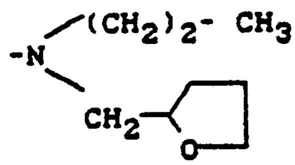 | $n_D^{20}$: 1,4760 |
| 108 | O | H | $-NH-CH_2-CH(C_2H_5)_2$ | $n_D^{20}$: 1,4666 |
| 109 | O | H | $-NH(CH_2)_3-OC_2H_5$ | $n_D^{20}$: 1,4678 |
| 110 | O | H | 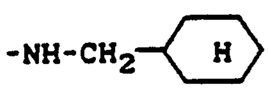 | $n_D^{20}$:1,4836 |

| Bsp. Nr. | X | R | $-N<^{R^1}_{R^2}$ | physikalische Eigenschaften |
|---|---|---|---|---|
| 111 | O | H | $-NH-CH_2-CH(CH_3)_2$ | $n_D^{20}$: 1,4643 |
| 112 | O | $C_2H_5$ | 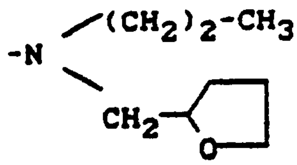 x 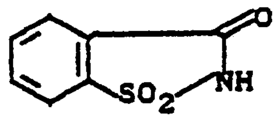 | $n_D^{20}$: 1,5226 |
| 113 | S | H | 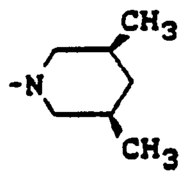 | $n_D^{20}$: 1,4900 |
| 114 | S | H | 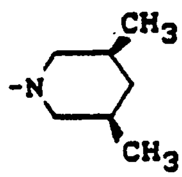 | $n_D^{20}$: 1,5000 |
| 115 | S | H | 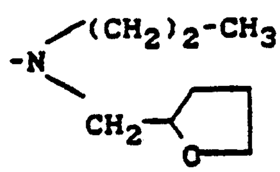 | $n_D^{20}$: 1,5030 |
| 116 | S | H | $-NH-(CH_2)_3OC_2H_5$ | $n_D^{20}$: 1,4913 |
| 117 | S | H | $-NH-CH_2-CH(C_2H_5)_2$ | $n_D^{20}$: 1,4910 |

| Bsp. Nr. | X | R | 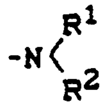 | physikalische Eigenschaften |
|---|---|---|---|---|
| 118 | S | H | 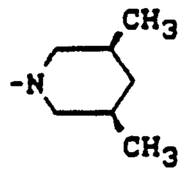 | $n_D^{20}$: 1,5179 |
| | | | x 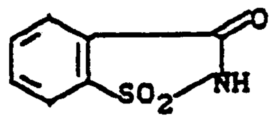 | |
| 119 | O | H | $-NH-(CH_2)_3-OC_2H_5$ | $n_D^{20}$: 1,5058 |
| | | | x 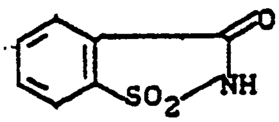 | |
| 120 | S | H | 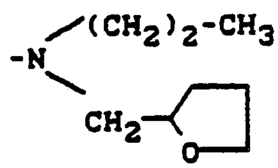 | $n_D^{20}$: 1,5260 |
| | | | x 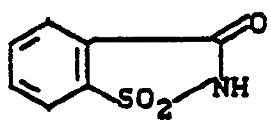 | |
| 121 | S | H | $-NH(CH_2)_3OC_2H_5$ | $n_D^{20}$: 1,5318 |
| | | | x 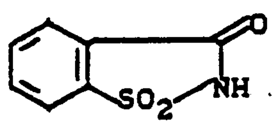 | |
| 122 | S | H | $-NH-CH_2-CH(C_2H_5)_2$ | $n_D^{20}$: 1,5272 |
| | | | x 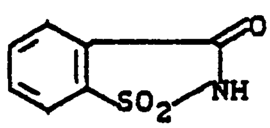 | |

| Bsp. Nr. | X | R | 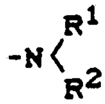 | physikalische Eigenschaften |
|---|---|---|---|---|
| 123 | O | H | 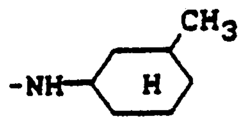 | $n_D^{20}$: 1,4808 |
| 124 | S | H | 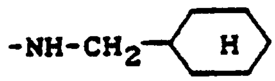 | $n_D^{20}$: 1,5056 |
| 125 | O | H | 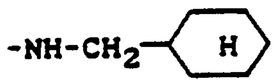 x 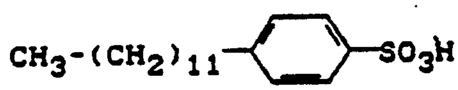 | $^1$H-NMR*): 4,5-4,6 (m) 4,0-4,1 (m) 3,6-3,7 (m) 3,3-3,5 (m) |
| 126 | O | $CH_3$ | 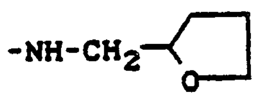 | $n_D^{20}$: 1,4812 |
| 127 | S | 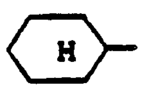 | 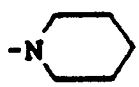 | $n_D^{20}$: 1,5198 |
| 128 | S | 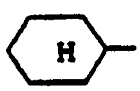 | 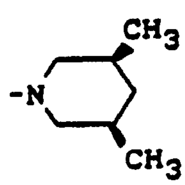 | $n_D^{20}$: 1,5065 |
| 129 | S | 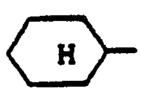 | 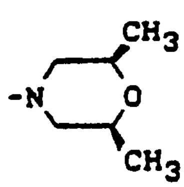 | $n_D^{20}$: 1,5147 |

| Bsp. Nr. | X | R | 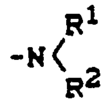 | physikalische Eigenschaften |
|---|---|---|---|---|
| 130 | S | 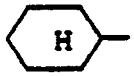 | 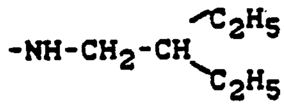 | $n_D^{20}$: 1,5033 |
| 131 | S | 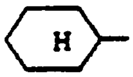 | 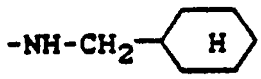 | $n_D^{20}$: 1,5186 |
| 132 | S | 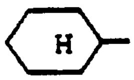 | 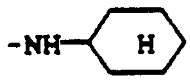 | $n_D^{20}$: 1,5242 |
| 133 | S | 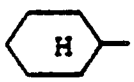 | 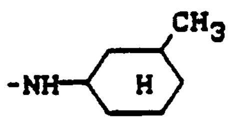 | $n_D^{20}$: 1,5189 |
| 134 | S | 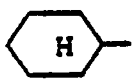 | 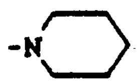 | $^1H$-NMR*): 4,6–4,8 (m)<br>3,0–3,7 (m) |
| | | | x 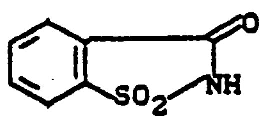 | |
| 135 | S | 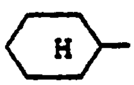 | 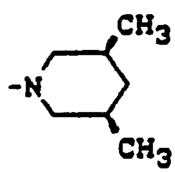 | $^1H$-NMR*): 4,5–4,7 (m)<br>2,7–3,7 (m) |
| 136 | S | 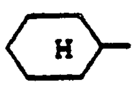 | 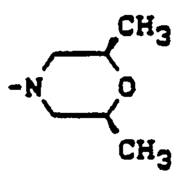 | $^1H$-NMR*): 4,6–4,9 (m)<br>4,1–4,3 (m)<br>3,0–3,9 (m) |
| | | | x 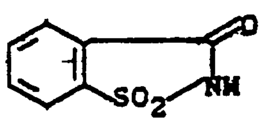 | |

| Bsp. Nr. | X | R | $-N{<}{R^1 \atop R^2}$ | physikalische Eigenschaften |
|---|---|---|---|---|
| 137 | S | 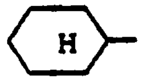 | 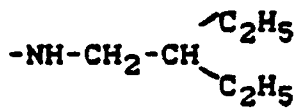 | $^1H$-NMR*): 4,6-4,7 (m)<br>3,5-3,6 (m)<br>2,7-3,3 (m) |
| 138 | S | 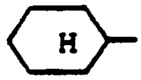 | 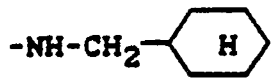 | $^1H$-NMR*): 4,6-4,7 (m)<br>3,5-3,7 (m)<br>2,7-3,3 (m) |
| | | | x 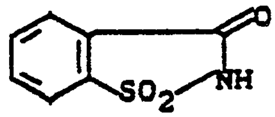 | |
| 139 | S | 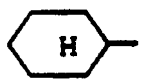 | 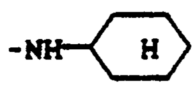 | $^1H$-NMR*): 4,6-4,8 (m)<br>3,1-3,6 (m)<br>2,7-2,9 (m) |
| 140 | S | 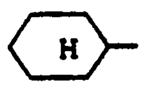 | 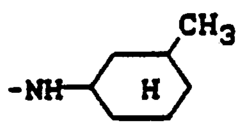 | $^1H$-NMR*): 4,6-4,8 (m)<br>3,1-3,6 (m)<br>2,7-2,9 (m) |
| | | | x 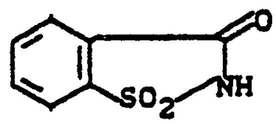 | |
| 141 | O | H | $-NH-CH_2-CH(OC_2H_5)_2$ | $n_D^{20}$: 1,4120 |
| 142 | O | $CH_3$ | 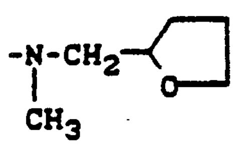 | $n_D^{20}$: 1,5050 |
| 143 | O | $CH_3$ | 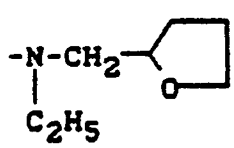 | $n_D^{20}$: 1,4803 |

| Bsp. Nr. | X | R | 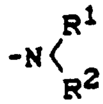 | physikalische Eigenschaften |
|---|---|---|---|---|
| 144 | O | H | 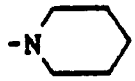 | $n_D^{20}$: 1,480 |
| 145 | O | H | 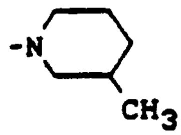 | $n_D^{20}$: 1,479 |
| 146 | O | H | 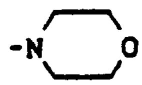 | $n_D^{20}$: 1,482 |
| 147 | O | $CH_3$ | 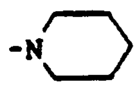 | $n_D^{20}$: 1,484 |
| 148 | O | $CH_3$ | 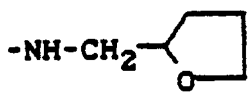 | $n_D^{20}$: 1,5221 |
| 149 | O | $CH_3$ | 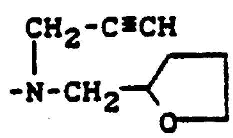 | $n_D^{20}$: 1,4924 |
| 150 | S | $CH_3$ | 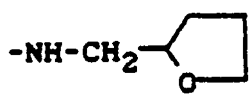 | $n_D^{20}$: 1,5070 |
| 151 | S | $CH_3$ | 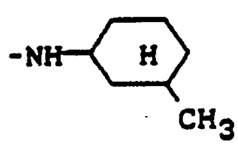 | $n_D^{20}$: 1,5030 |

| Bsp. Nr. | X | R | 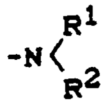 | physikalische Eigenschaften |
|---|---|---|---|---|
| 152 | O | $CH_3$ | 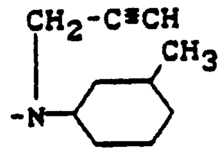 | $n_D^{20}$: 1,4915 |
| 153 | S | $CH_3$ | 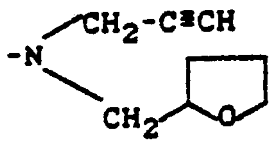 | $n_D^{20}$: 1,5131 |
| 154 | O | $CH_3$ | $-NH-C(CH_3)_3$ | $n_D^{20}$: 1,4686 |
| 155 | O | H | $-NH-C(CH_3)_3$ | $n_D^{20}$: 1,4632 |
| 156 | S | $CH_3$ | 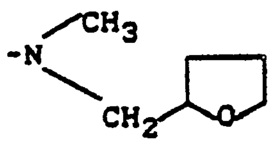 | $n_D^{20}$: 1,5079 |
| 157 | S | $CH_3$ | 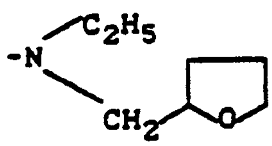 | $n_D^{20}$: 1,5052 |
| 158 | S | $CH_3$ | $-NH-C(CH_3)_3$ | Fp. 68° -72° C |
| 159 | S | H | 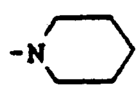 | $n_D^{20}$: 1,5030 |
| 160 | S | H | 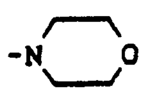 | $n_D^{20}$: 1,508 |

| Bsp. Nr. | X | R | 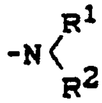 | physikalische Eigenschaften |
|---|---|---|---|---|
| 161 | S | H | 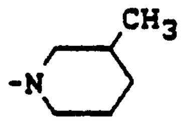 | x 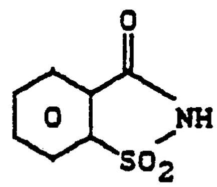 |
| 162 | S | H | 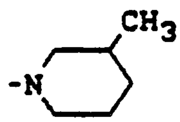 | $n_D^{20}$: 1.501 |
| 163 | S | $C_2H_5$ | 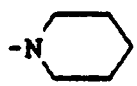 | $n_D^{20}$: 1.507 |
| 164 | S | $C_2H_5$ | 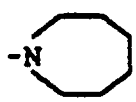 | $n_D^{20}$: 1.511 |
| 165 | S | $C_2H_5$ | 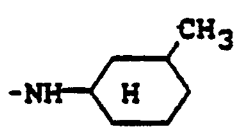 | $n_D^{20}$: 1.503 |
| 166 | S | $C_2H_5$ | 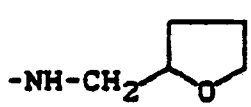 | $n_D^{20}$: 1.501 |
| 167 | O | $C_2H_5$ | 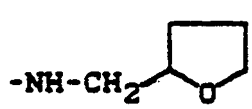 | $n_D^{20}$: 1.484 |

| Bsp. Nr. | X | R | 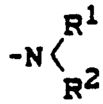 | physikalische Eigenschaften |
|---|---|---|---|---|
| 168 | S | H | 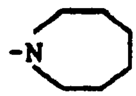 | $n_D^{20}$: 1.501 |
| 169 | O | $CH_3$ | 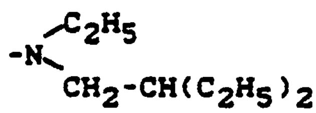 | $n_D^{20}$: 1.4674 |
| 170 | O | $CH_3$ | 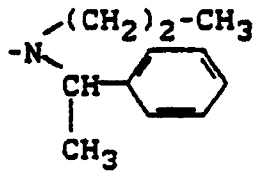 | $n_D^{20}$: 1.5043 |
| 171 | O | $CH_3$ | 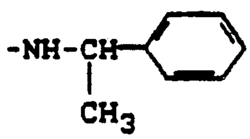 | $n_D^{20}$: 1.5088 |
| 172 | O | $-CH_2-C(CH_3)_3$ | $-NH-CH_3$ | Kp. 140° C/ 0.5 mbar |
| 173 | O | $-CH_2-C(CH_3)_3$ | $-NH-C_2H_5$ | Kp.143-145° C/ 0.5 mbar |
| 174 | O | 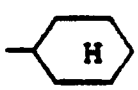 | $-NH-CH_3$ | Kp. 172° C/ 0.9 mbar |
| 175 | O | 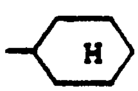 | $-NH-C_2H_5$ | Kp. 178° C/ 0.7 mbar |

| Bsp. Nr. | X | R | $-N\langle^{R^1}_{R^2}$ | physikalische Eigenschaften |
|---|---|---|---|---|
| 176 | O | 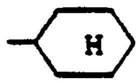 | $-N$ $CH_3$<br>$(CH_2)_2-CH_3$ | $n_D^{20}$: 1.4898 |
| 177 | O | 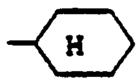 | $-N$ $CH_3$<br>$(CH_2)_3-CH_3$ | $n_D^{20}$: 1.4893 |
| 178 | O | 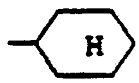 | $-N$ $C_2H_5$<br>$(CH_2)_2-CH_3$ | $n_D^{20}$: 1.4885 |
| 179 | O | 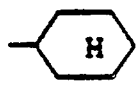 | $-N$ $C_2H_5$<br>$(CH_2)_3-CH_3$ | $n_D^{20}$: 1.4874 |
| 180 | O | $-CH_2-C(CH_3)_3$ | $-N$ $CH_3$<br>$(CH_2)_2-CH_3$ | $n_D^{20}$: 1.4722 |
| 181 | O | $-CH_2-C(CH_3)_3$ | $-N$ $CH_3$<br>$(CH_2)_3-CH_3$ | $n_D^{20}$: 1.4728 |
| 182 | O | $-CH_2-C(CH_3)_3$ | $-N$ $C_2H_5$<br>$(CH_2)_2-CH_3$ | $n_D^{20}$: 1.4732 |
| 183 | O | $-CH_2-C(CH_3)_3$ | $-N$ $C_2H_5$<br>$(CH_2)_3-CH_3$ | $n_D^{20}$: 1.4726 |

| Bsp. Nr. | X | R | $-N\langle{R^1 \atop R^2}$ | physikalische Eigenschaften |
|---|---|---|---|---|
| 184 | O | $CH_3$ | $-NH-(CH_2)_2-CH_3$ | $n_D^{20}$: 1.4684 |
| 185 | O | $-CH_2-C(CH_3)_3$ | 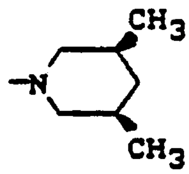 | $n_D^{20}$: 1.4831 (Diastereomer A) |
| 186 | O | 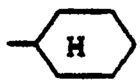 | 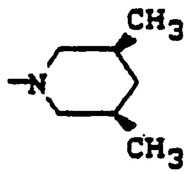 | Fp.68-70° C (Diastereomer A) |
| 187 | O | 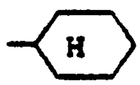 | 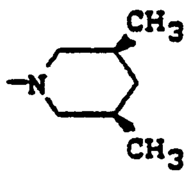 | $n_D^{20}$: 1.4959 (Diastereomer B) |
| 188 | O | $CH_3$ | $-N$ $CH_2-COOC_2H_5$<br>$CH_2-CH(C_2H_5)_2$ | $n_D^{20}$: 1.4668 |
| 189 | O | $CH_3$ | $-N$ $C_2H_5$<br>$(CH_2)_3-OC_2H_5$ | $n_D^{20}$: 1.4656 |

Anwendungsbeispiele

Im den folgenden Anwendungsbeispiel wurden die nachstehend aufgeführten Verbindungen als Vergleichsubstanzen eingesetzt:

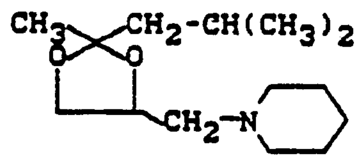

(A)

2-Isobutyl-2-methyl-4-(1-piperidinylmethyl)-1,3-dioxolan und

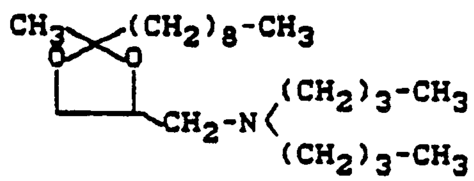

(B)

2-Methyl-2-nonyl-4-di-n-butylaminomethyl-1,3-dioxolan

(beide bekannt aus EP 97 822).

Beispiel A

Pyrenophora teres-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 1, 2, 3, 4, 5, 6, 7 und 8.

## Patentansprüche

1. Aminomethylheterocyclen der allgemeinen Formel (I),

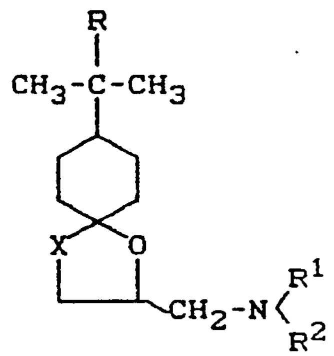

(I)

in welcher

X für Sauerstoff oder Schwefel steht,

R für Wasserstoff, für geradkettiges oder verzweigtes Alkyl mit 1 bis 6 Kohlenstoffatomen oder für jeweils gegebenenfalls ein- bis dreifach, gleich oder verschieden durch Alkyl mit 1 bis 4 Kohlenstoffatomen und/oder Halogen substituiertes Phenyl oder Cyclohexyl steht und

$R^1$ und $R^2$ unabhängig voneinander jeweils für Wasserstoff; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls in Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:
Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen:
Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten kann, wobei als Substituenten infrage kommen: jeweils geradkettiges cder verzweigtes Alkyl oder Hydroxyalkyl mit jeweils 1 bis 4 Kohlenstoffatomen,

deren Säureadditionssalze, deren geometrische und optische Isomere und Isomerengemische.

**2.** Aminomethylheterocyclen gemäß Anspruch 1, wobei in der Formel (I)

X für Sauerstoff oder Schwefel steht,

R für Wasserstoff, Methyl, Ethyl, Neopentyl, Cyclohexyl oder Phenyl steht und

$R^1$ und $R^2$ unabhängig voneinander jeweils für Wasserstoff, Methyl, Ethyl, n- oder i-Propyl, n-, i-, s- oder t-Butyl, n- oder i-Pentyl, n-oder i-Hexyl, n- oder i-Heptyl, n- oder i-Octyl, Allyl,

n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Dioxanylethyl, Oxolanylmethyl, Oxolanylethyl, für jeweils gegebenenfalls ein- bis fünffach, gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

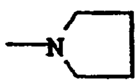 ; 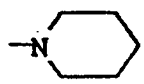 ; 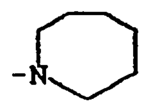 ; 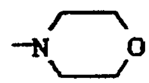

oder 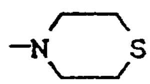

stehen, wobei als Substituenten infrage kommen: Methyl, Ethyl oder Hydroxymethyl, deren Säureadditionssalze, deren geometrische und optische Isomere und deren Isomerengemische.

3. Aminomethylheterocyclen gemäß Anspruch 1, wobei in der Formel (I)

X für Sauerstoff oder Schwefel steht,

R für Wasserstoff, Methyl oder Ethyl steht und

$R^1$ und $R^2$ unabhängig voneinander jeweils für Wasserstoff, Methyl, Ethyl, n- oder i-Propyl, n-, i-, s-oder t-Butyl, n- oder i-Pentyl, n-oder i-Hexyl, Allyl, n- oder i-Butenyl, n-oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Methoxycarbonylmethyl, Methoxycarbonylethyl, Methoxycarbonylpropyl, Ethoxycarbonylmethyl, Ethoxycarbonylethyl, Ethoxycarbonylethyl, Ethoxycarbonylpropyl, Propoxycarbonylmethyl, Propoxycarbonylethyl, Propoxycarbonylpropyl, Dioxolanylmethyl, Dioxolanylethyl, Dioxanylmethyl, Dioxanylethyl, Oxolanylmethyl, Oxolanylethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl steht oder

$R^1$ und $R^2$ gemeinsam mit dem Stickstoffatom, an welches sie gebunden sind, für einen gegebenenfalls ein-bis dreifach, gleich oder verschieden substituierten Heterocyclus der Formel

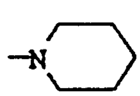 ; 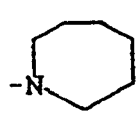 ; 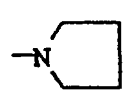 oder 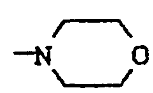

stehen,

wobei als Substituenten infrage kommen: Methyl, Ethyl, Hydroxymethyl, deren Säureadditionssalze,

deren geometrische und optische Isomere und deren Isomerengemische.

**4.** Verfahren zur Herstellung von Aminomethylheterocyclen der allgemeinen Formel (I),

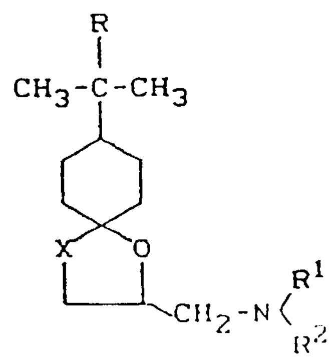

(I)

in welcher

X für Sauerstoff oder Schwefel steht,

R für Wasserstoff, für geradkettiges oder verzweigtes Alkyl mit 1 bis 6 Kohlenstoffatomen oder für jeweils gegebenenfalls ein- bis dreifach, gleich oder verschieden durch Alkyl mit 1 bis 4 Kohlenstoffatomen und/oder Halogen substituiertes Phenyl oder Cyclohexyl steht und

$R^1$ und $R^2$ unabhängig voneinander jeweils für Wasserstoff; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw. Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil oder für jeweils gegebenenfalls in Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil stehen, wobei als Substituenten jeweils infrage kommen:
Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen:
Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder Verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen,

sowie deren Säureadditionssalze, deren geometrische und optische Isomere und Isomerengemische, dadurch gekennzeichnet, daß man

(a) substituierte Heterocyclen der Formel (II),

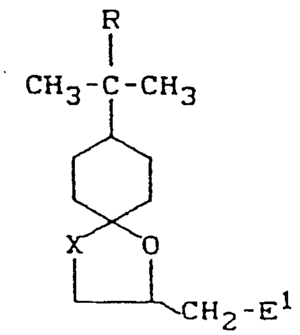

(II)

in welcher
R und X die oben angegebene Bedeutung haben und
$E^1$ für eine elektronenanziehende Abgangsgruppe steht,
mit Aminen der Formel (III),

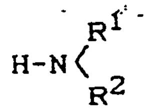

(III)

in welcher
$R^1$ und $R^2$ die oben angegebene Bedeutung haben,
gegebenenfalls in Gegenwart eines Verdünnungsmittels und gegebenenfalls in Gegenwart eines Säurebindemittels umsetzt, oder
(b) die nach Verfahren (a) erhältlichen Aminomethylheterocyclen der Formel (Ia),

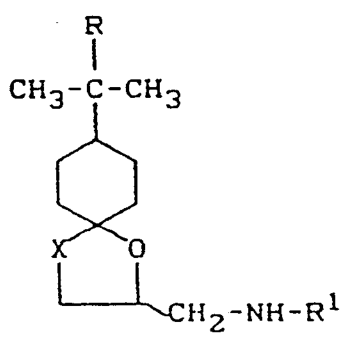

(Ia)

in welcher
R, $R^1$ und X die oben angegbene Bedeutung haben,
mit Alkylierungsmitteln der Formel (IV),

$R^{2-1}$-$E^2$ (IV)

in welcher
$R^{2-1}$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit 1 bis 6 Kohlenstoffatomen oder Hydroxyalkoxyalkyl mit 2 bis 6 Kohlenstoffatomen in den einzelnen Alkylteilen, für Alkoxycarbonylalkyl mit 1 bis 6 Kohlenstoffatomen je Alkoxy- bzw Alkylteil, für jeweils geradkettiges oder verzweigtes Dioxolanylalkyl, Dioxanylalkyl oder Oxolanylalkyl mit jeweils 1 bis 4 Kohlenstoffatomen im Alkylteil, für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis

4 Kohlenstoffatomen im geradkettigen oder verzweigten Alkylteil, wobei als Substituenten jeweils infrage kommen: Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl- bzw. Alkenylteil, wobei als Arylsubstituenten jeweils infrage kommmen: Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen

und

$E^2$ für eine elektronenanziehende Abgangsgruppe steht,

gegebenenfalls in Gegenwart eines Verdünnungsmittels und gegebenenfalls in Gegenwart eines Säurebindemittels umsetzt und gegebenenfalls anschließend eine Säure addiert oder eine physikalische Trennmethode anschließt.

5. Schädlingsbekämpfungsmittel, gekennzeichnet durch einen Gehalt an mindestens einem Aminomethylheterocyclus der Formel (I) nach den Ansprüchen 1 und 4

6. Verwendung von Aminomethylheterocyclen der Formel (I) nach den Ansprüchen 1 und 4 zur Bekämpfung von Schädlingen.

7. Verfahren zur Bekämpfung von Schädlingen, dadurch gekennzeichnet, daß man Aminomethylheterocyclen der Formel (I) nach den Ansprüchen 1 und 4 auf Schädinge und/oder ihren Lebensraum einwirken läßt.

8. Verfahren zur Herstellung von Schädingsbekämpfungsmitteln, dadurch gekennzeichnet, daß man Aminomethylheterocyclen der Formel (I) nach den Ansprüchen 1 und 4 mit Streckmitteln und/oder oberflächenaktiven Mitteln vermischt.

## Claims

1. Aminomethyl heterocyclic compounds of the general formula (I)

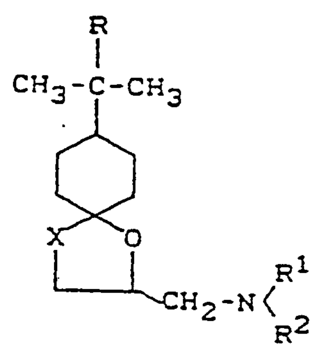

(I)

in which

X represents oxygen or sulphur,

R represents hydrogen, or represents straight-chain or branched alkyl with 1 to 6 carbon atoms, or represents phenyl or cyclohexyl, in each case optionally mono-, di- or trisubstituted by indentical or different substituents from the group comprising alkyl with 1 to 4 carbon atoms and/or halogen, and

$R^1$ and $R^2$ independently of one another each represent hydrogen; or represent in each case straight-chain or branched alkyl with 1 to 12 carbon atoms, alkenyl with 3 to 8 carbon atoms, alkinyl with 3 to 8 carbon atoms, hydroxyalkyl with 2 to 6 carbon atoms,

alkoxyalkyl or dialkoxyalkyl with in each case 1 to 6 carbon atoms or hydroxyalkoxyalkyl with 2 to 6 carbon atoms in the individual alkyl parts or represent alkoxycarbonylalkyl with 1 to 6 carbon atoms in the alkoxy and alkyl part, or represent in each case straight-chain or branched dioxolanylalkyl or dioxanylalkyl or oxolanylalkyl with in each case 1 to 4 carbon atoms in the alkyl part, or represent cycloalkyl or cycloalkylalkyl with in each case 3 to 7 carbon atoms in the cycloalkyl part and where appropriate 1 to 4 carbon atoms in the straight-chain or branched alkyl part and in each case optionally mono- or polysubstituted in the cycloalkyl part by identical or different substituents, possible substituents in each case being:
halogen and in each case straight-chain or branched alkyl, alkoxy, halogenoalkyl and halogenoalkoxy with in each case 1 to 4 carbon atoms and where appropriate 1 to 9 identical or different halogen atoms; or furthermore represent arylalkyl, arylalkenyl or aryl with in each case 6 to 10 carbon atoms in the aryl part and where appropriate up to 6 carbon atoms in the straight-chain or branched alkyl or alkenyl part, in each case optionally mono- or polysubstituted in the aryl part by identical or different substituents, possible substituents on the aryl in each case being:
halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and where appropriate 1 to 9 identical or different halogen atoms, or

$R^1$ and $R^2$, together with the nitrogen atom to which they are bonded, represent a saturated 5- to 7-membered heterocyclic radical which can optionally contain a further hetero atom, in particular nitrogen, oxygen or sulphur, and is optionally mono- or polysubstituted by identical or different substituents, possible substituents being:
in each case straight-chain or branched alkyl and hydroxyalkyl with in each case 1 to 4 carbon atoms,
acid addition salts thereof and geometric and optical isomers and isomer mixtures thereof.

**2.** Aminomethyl heterocyclic compounds according to Claim 1,

wherein, in formula (I),

X represents oxygen or sulphur,

R represents hydrogen, methyl, ethyl, neopentyl, cyclohexyl or phenyl and

$R^1$ and $R^2$ independently of one another in each case represent hydrogen, methyl, ethyl, n- or i-propyl, n-, i-, s- or t-butyl, n- or i-pentyl, n-or i-hexyl, n- or i-heptyl, n- or i-octyl, allyl, n- or i-butenyl, n- or i-pentenyl, propargyl, n-or i-butinyl, hydroxyethyl, hydroxypropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl, dioxolanylmethyl, dioxolanylethyl, dioxanylmethyl, dioxanylethyl, oxolanylmethyl or oxolanylethyl, or represent cyclopropyl, cyclopropylmethyl, cyclopropylethyl, cyclopropylpropyl, cyclopentyl, cyclopentylmethyl, cyclohexyl or cyclohexylmethyl, in each case optionally mono-, di-, tri-, tetra- or pentasubstituted by identical or different substituents from the group comprising fluorine, chlorine, bromine, methyl, ethyl, n- or i-propyl and/or n-, i-, s- or t-butyl, or represent phenyl, benzyl or phenethyl in each case optionally mono-, di- or trisubstituted by identical or different substituents, possible substituents in each case being:
fluorine, chlorine, bromine, cyano, nitro, methyl, ethyl, n- or i-propyl, n-, i-, s- or t-butyl, methoxy, ethoxy, methylthio, trifluoromethyl, trifluoromethoxy, trifluoromethylthio, methoxycarbonyl, ethoxycarbonyl and methoximinomethyl, or

$R^1$ and $R^2$, together with the nitrogen atom to which they are bonded, represent a heterocyclic radical of the formula

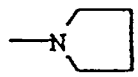 ; 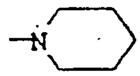 ; 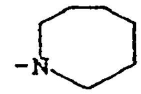 ; 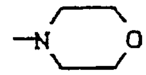

or 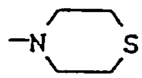

which is optionally mono-, di- or trisubstituted by identical or different substituents, possible substituents being:
methyl, ethyl and hydroxymethyl, acid addition salts thereof, geometric and optical isomers thereof and isomer mixtures thereof.

3. Aminomethyl heterocyclic compounds according to Claim 1, wherein, in formula (I),

X represents oxygen or sulphur,

R represents hydrogen, methyl or ethyl and

$R^1$ and $R^2$ independently of one another each represent hydrogen, methyl, ethyl, n- or i-propyl, n-, i-, s-or t-butyl, n- or i-pentyl, n- or i-hexyl, allyl, n- or i-butenyl, n- or i-pentenyl, propargyl, n- or i-butinyl, hydroxyethyl, hydroxypropyl, methoxyethyl, methoxypropyl, ethoxyethyl, ethoxypropyl, hydroxyethoxyethyl, dimethoxyethyl, diethoxyethyl, methoxycarbonylmethyl, methoxycarbonylethyl, methoxycarbonylpropyl, ethoxycarbonylmethyl, ethoxycarbonylethyl, ethoxycarbonylpropyl, propoxycarbonylmethyl, propoxycarbonylethyl, propoxycarbonylpropyl, dioxolanylmethyl , dioxolanylethyl, dioxanylmethyl, dioxanylethyl, oxolanylmethyl, oxolanylethyl, cyclopropylmethyl, dichlorocyclopropylmethyl dimethylcyclopropylmethyl, dichlorodimethylcyclopropylmethyl, cyclopentyl, cyclohexyl or cyclohexylmethyl, or

$R^1$ and $R^2$, together with the nitrogen atom to which they are bonded, represent a heterocyclic radical of the formula

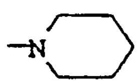 ; 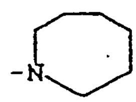 ; 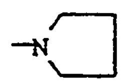 or 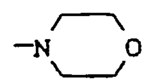

which is optionally mono-, di- or trisubstituted by identical or different substituents, possible substituents being: methyl, ethyl and hydroxymethyl,

acid addition salts thereof, geometric and optical isomers thereof and isomer mixtures thereof.

4. Process for the preparation of aminomethyl heterocyclic compounds of the general formula (I)

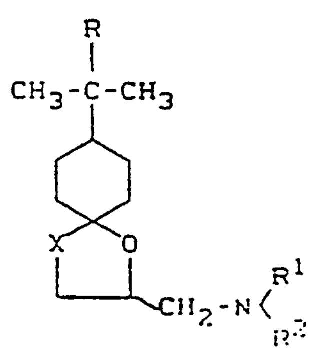

(I)

in which

X represents oxygen or sulphur,

R represents hydrogen, or represents straight-chain or branched alkyl with 1 to 6 carbon atoms, or represents phenyl or cyclohexyl, in each case optionally mono-, di- or trisubstituted by indentical or different substituents from the group comprising alkyl with 1 to 4 carbon atoms and/or halogen, and

$R^1$ and $R^2$ independently of one another each represent hydrogen; or represent in each case straight-chain or branched alkyl with 1 to 12 carbon atoms, alkenyl with 3 to 8 carbon atoms, alkinyl with 3 to 8 carbon atoms, hydroxyalkyl with 2 to 6 carbon atoms, alkoxyalkyl or dialkoxyalkyl with in each case 1 to 6 carbon atoms or hydroxyalkoxyalkyl with 2 to 6 carbon atoms in the individual alkyl parts or represent alkoxycarbonylalkyl with 1 to 6 carbon atoms in the alkoxy and alkyl part, or represent in each case straight-chain or branched dioxolanylalkyl or dioxanylalkyl or oxolanylalkyl with in each case 1 to 4 carbon atoms in the alkyl part, or represent cycloalkyl or cycloalkylalkyl with in each case 3 to 7 carbon atoms in the cycloalkyl part and where appropriate 1 to 4 carbon atoms in the straight-chain or branched alkyl part and in each case optionally mono- or polysubstituted in the cycloalkyl part by identical or different substituents, possible substituents in each case being:
halogen and in each case straight-chain or branched alkyl, alkoxy, halogenoalkyl and halogenoalkoxy with in each case 1 to 4 carbon atoms and where appropriate 1 to 9 identical or different halogen atoms; or furthermore represent arylalkyl, arylalkenyl or aryl with in each case 6 to 10 carbon atoms in the aryl part and where appropriate up to 6 carbon atoms in the straight-chain or branched alkyl or alkenyl part, in each case optionally mono- or polysubstituted in the aryl part by identical or different substituents, possible substituents on the aryl in each case being:
halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and where appropriate 1 to 9 identical or different halogen atoms, or

$R^1$ and $R^2$, together with the nitrogen atom to which they are bonded, represent a saturated 5- to 7-membered heterocyclic radical which can optionally contain a further hetero atom, in particular nitrogen, oxygen or sulphur, and is optionally mono- or polysubstituted by identical or different substituents, possible substituents being:
in each case straight-chain or branched alkyl and hydroxyalkyl with in each case 1 to 4 carbon atoms,

acid addition salts thereof and geometric and optical isomers and isomer mixtures thereof, characterised in that

(a) substituted heterocyclic compounds of the formula (II)

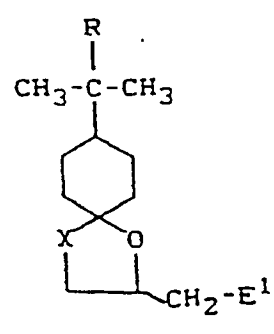

(II)

in which

R and X have the abovementioned meaning and

$E^1$ represents an electron-withdrawing leaving group,

are reacted with amines of the formula (III)

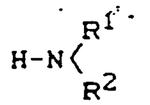

(III)

in which

$R^1$ and $R^2$ have the abovementioned meaning,

if appropriate in the presence of a diluent and if appropriate in the presence of an acid-binding agent, or

(b) the aminomethyl heterocyclic compounds obtainable by process (a), of the formula (Ia)

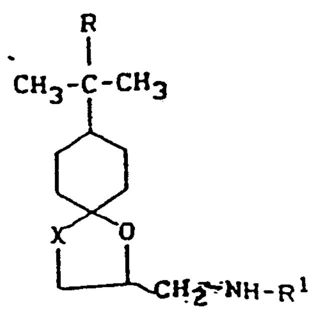

(Ia)

in which

R, $R^1$ and X have the abovementioned meaning,

are reacted with alkylating agents of the formula (IV)

$R^{2-1}$-$E^2$ (IV)

in which

$R^{2-1}$ represents in each case straight-chain or branched alkyl with 1 to 12 carbon atoms, alkenyl with 3 to 8 carbon atoms, alkinyl with 3 to 8 carbon atoms, hydroxyalkyl with 2 to 6 carbon atoms, alkoxyalkyl or dialkoxyalkyl with 1 to 6 carbon atoms or hydroxyalkoxyalkyl with 2 to 6 carbon atoms in the individual alkyl parts, or represents alkoxycarbonylalkyl with 1 to 6 carbon atoms in each alkoxy and alkyl part, or represents in each case straight-chain or branched dioxolanylalkyl, dioxanylalkyl or oxolanylalkyl with in each case 1 to 4 carbon atoms in the alkyl part, or represents cycloalkyl or cycloalkylalkyl with in each case 3 to 7 carbon atoms in the cycloalkyl part and where appropriate 1 to 4 carbon atoms in the straight-chain or branched alkyl part and in each case optionally mono- or polysubstituted in the cycloalkyl part by identical or different substituents, possible substituents in each case being: halogen and in each case straight-chain or branched alkyl, alkoxy, halogenoalkyl and halogenoalkoxy with in each case 1 to 4 carbon atoms and where appropriate 1 to 9 identical or different halogen atoms; or furthermore represents arylalkyl or arylalkenyl with in each case 6 to 10 carbon atoms in the aryl part and up to 6 carbon atoms in the straight-chain or branched alkyl or alkenyl part, in each case optionally mono- or polysubstituted in the aryl part by identical or different substituents, possible substituents on the aryl in each case being: halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and where appropriate 1 to 9 identical or different halogen atoms, and

$E^2$ represents an electron-withdrawing leaving group,

if appropriate in the presence of a diluent and if appropriate in the presence of an acid-binding agent, and, if appropriate, an acid is then added on or the reaction is followed by a physical separation method.

**5.** Agents for combating pests, characterised in that they contain at least one aminomethyl heterocyclic

compound of the formula (I) according to Claims 1 and 4.

6. Use of aminomethyl heterocyclic compounds of the formula (I) according to Claims 1 and 4 for combating pests.

7. Method of combating pests, characterised in that aminomethyl heterocyclic compounds of the formula (I) according to Claims 1 and 4 are allowed to act on pests and/or their environment.

8. Process for the preparation of agents for combating pests, characterised in that aminomethyl heterocyclic compounds of the formula (I) according to Claims 1 and 4 are mixed with extenders and/or surface-active agents.

**Revendications**

1. Composés hétérocycliques aminométhylés répondant à la formule générale (I)

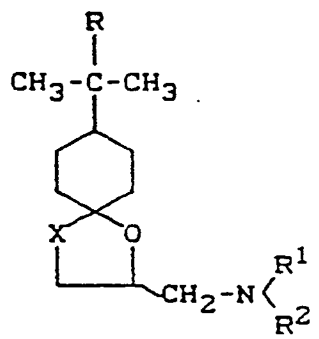

(I)

dans laquelle

X représente un atome d'oxygène ou un atome de soufre,

R représente un atome d'hydrogène, un groupe alkyle à chaîne droite ou ramifiée et contenant de 1 à 6 atomes de carbone ou un groupe phényle ou cyclohexyle, chaque fois éventuellement substitué une à trois fois, de manière identique ou différente, par un groupe alkyle contenant de 1 à 4 atomes de carbone et/ou par un atome d'halogène et

$R^1$ et $R^2$ représentent chacun, indépendamment l'un de l'autre, un atome d'hydrogène ; un groupe alkyle contenant de 1 à 12 atomes de carbone, un groupe alcényle contenant de 3 à 8 atomes de carbone, un groupe alcynyle contenant de 3 à 8 atomes de carbone, un groupe hydroxyalkyle contenant de 2 à 6 atomes de carbone, un groupe alcoxyalkyle ou dialcoxyalkyle contenant chacun de 1 à 6 atomes de carbone ou un groupe hydroxyalcoxyalkyle contenant de 2 à 6 atomes de carbone dans les fractions alkyle individuelles, ces groupes étant chacun à chaîne droite ou ramifiée ; un groupe alcoxycarbonylalkyle contenant de 1 à 6 atomes de carbone, chaque fois, dans la fraction alcoxy ou alkyle, un groupe dioxolanylalkyle, un groupe dioxanylalkyle ou un groupe oxolanylalkyle, chaque fois à chaîne droite ou ramifiée, contenant chacun de 1 à 4 atomes de carbone dans la fraction alkyle, ou un groupe cycloalkyle ou cycloalkylalkyle, chaque fois éventuellement substitué de manière identique ou différente, de une à plusieurs fois, dans la fraction alkyle, contenant chacun de 3 à 7 atomes de carbone dans la fraction cycloalkyle et éventuellement 1 à 4 atomes de carbone dans la fraction alkyle à chaîne droite ou ramifiée, groupes dans lesquels, comme substituants, on peut chaque fois envisager :

un atome d'halogène, un groupe alkyle, un groupe alcoxy, un groupe halogénalkyle ou un groupe halogénalcoxy, chaque fois à chaîne droite ou ramifiée, contenant chacun de 1 à 4 atomes de carbone et éventuellement 1 à 9 atomes d'halogène identiques ou différents ; en outre, un groupe arylalkyle, un groupe arylalcényle ou un groupe aryle, chaque fois éventuellement substitué une à plusieurs fois, de manière identique ou différente dans la fraction aryle, contenant chacun de 6 à 10 atomes de carbone dans

la fraction aryle et éventuellement jusqu'à 6 atomes de carbone dans la fraction alkyle ou alcényle à chaîne droite ou ramifiée, dans lesquels, comme substituants du radical aryle, on peut chaque fois envisager :
un atome d'halogène, un groupe cyano, un groupe nitro, un groupe alkyle, un groupe alcoxy, un groupe alkylthio, un groupe halogénalkyle, un groupe halogénalcoxy, un groupe halogénalkylthio, un groupe alcoxycarbonyle ou un groupe alcoximinoalkyle chaque fois à chaîne droite ou ramifiée, contenant chacun de 1 à 4 atomes de carbone dans les fractions alkyle individuelles et éventuellement 1 à 9 atomes d'halogène identiques ou différents ou

$R^1$ et $R^2$ représentent, conjointement avec l'atome d'azote auquel ils sont liés, un hétérocycle penta- à heptagonal saturé, éventuellement substitué de une à plusieurs fois de manière identique ou différente, qui peut contenir éventuellement un autre hétéroatome, en particulier un atome d'azote, un atome d'oxygène ou un atome de soufre, dans lequel, comme substituants, on envisage : un groupe alkyle ou hydroxyalkyle chaque fois à chaîne droite ou ramifiée, contenant chacun de 1 à 4 atomes de carbone,

leurs sels d'addition d'acides, leurs isomères géométriques et optiques, ainsi que leurs mélanges d'isomères.

**2.** Composés hétérocycliques aminométhylés selon la revendication 1, dans lesquels, dans la formule (I),

X représente un atome d'oxygène ou un atome de soufre

R représente un atome d'hydrogène, un groupe méthyle, un groupe éthyle, un groupe néopentyle, un groupe cyclohexyle ou un groupe phényle ; et

$R^1$ et $R^2$ représentent chacun, indépendamment l'un de l'autre, un atome d'hydrogène, un groupe méthyle, un groupe éthyle, un groupe n- ou i-propyle, un groupe n-,i-,s- ou t-butyle, un groupe n- ou i-pentyle, un groupe n- ou i-hexyle, un groupe n- ou i-heptyle, un groupe n- ou i-octyle, un groupe allyle, un groupe n- ou i-butényle, un groupe n- ou i-pentényle, un groupe propargyle, un groupe n- ou i-butynyle, un groupe hydroxyéthyle, un groupe hydroxypropyle, un groupe méthoxyéthyle, un groupe éthoxyéthyle, un groupe propoxyéthyle, un groupe butoxyéthyle, un groupe méthoxypropyle, un groupe éthoxypropyle, un groupe propoxypropyle, un groupe butoxypropyle, un groupe hydroxyéthoxyéthyle, un groupe diméthoxyéthyle, un groupe diméthoxypropyle, un groupe diéthoxyéthyle, un groupe méthoxycarbonylméthyle, un groupe méthoxycarbonyléthyle, un groupe méthoxycarbonylpropyle, un groupe éthoxycarbonylméthyle, un groupe éthoxycarbonyléthyle, un groupe éthoxycarbonylpropyle, un groupe propoxycarbonylméthyle, un groupe propoxy-carbonyléthyle, un groupe propoxycarbonylpropyle, un groupe dioxolanylméthyle, un groupe dioxolanyléthyle, un groupe dioxanylméthyle, un groupe dioxanyléthyle, un groupe oxolanylméthyle, un groupe oxolanyléthyle, un groupe cyclopropyle, un groupe cyclopropylméthyle, un groupe cyclopropyléthyle, un groupe cyclopropylpropyle, un groupe cyclopentyle, un groupe cyclopentylméthyle, un groupe cyclohexyle ou un groupe cyclohexylméthyle, chacun éventuellement substitué de 1 à 5 fois, de manière identique ou différente, par un atome de fluor, un atome de chlore, un atome de brome, un groupe méthyle, un groupe éthyle, un groupe n- ou i-propyle, un groupe n-,i-,s- et/ou t-butyle, ou représentent un groupe phényle, un groupe benzyle ou un groupe phényléthyle, chacun éventuellement substitué de 1 à 3 fois, de manière identique ou différente, dans lesquels, comme substituants, on peut chaque fois envisager :
un atome de fluor, un atome de chlore, un atome de brome, un groupe cyano, un groupe nitro, un groupe méthyle, un groupe éthyle, un groupe n- ou i-propyle, un groupe n-,i-, s- ou t-butyle, un groupe méthoxy, un groupe éthoxy, un groupe méthylthio, un groupe trifluorométhyle, un groupe trifluorométhoxy, un groupe trifluorométhylthio, un groupe méthoxycarbonyle, un groupe éthoxycarbonyle ou un groupe méthoximinométhyle ou

$R^1$ et $R^2$ représentent, conjointement avec l'atome d'azote auquel ils sont liés, un hétérocycle chaque fois éventuellement substitué de 1 à 3 fois de manière identique ou différente, répondant aux formules

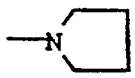 ; 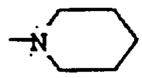 ; 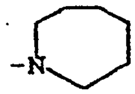 ; 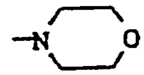

ou 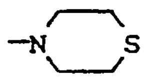

dans lesquelles, comme substituants, on envisage : un groupe méthyle, un groupe éthyle ou un groupe hydroxyméthyle, leurs sels d'addition d'acides, leurs isomères géométriques et optiques, ainsi que leurs mélanges d'isomères.

**3.** Composés hétérocycliques aminométhylés selon la revendication 1, dans lesquels, dans la formule (I),

X représente un atome d'oxygène ou un atome de soufre

R représente un atome d'hydrogène, un groupe méthyle ou un groupe éthyle et

$R^1$ et $R^2$ représentent chacun, indépendamment l'un de l'autre, un atome d'hydrogène, un groupe méthyle, un groupe éthyle, un groupe n- ou i-propyle, un groupe n-,i-,s- ou t-butyle, un groupe n- ou i-pentyle, un groupe n- ou i-hexyle, un groupe allyle, un groupe n- ou i-butényle, un groupe n- ou i-penténytle, un groupe propargyle, un groupe n- ou i-butynyle, un groupe hydroxyéthyle, un groupe hydroxypropyle, un groupe méthoxyéthyle, un groupe méthoxypropyle, un groupe éthoxyéthyle, un groupe éthoxypropyle, un groupe hydroxyéthoxyéthyle, un groupe diméthoxyéthyle, un groupe diéthoxyéthyle, un groupe méthoxycarbonylméthyle, un groupe méthoxycarbonyléthyle, un groupe méthoxycarbonylpropyle, un groupe éthoxycarbonylméthyle, un groupe éthoxycarbonyléthyle, un groupe éthoxycarbonylpropyle, un groupe propoxycarbonylméthyle, un groupe propoxycarbonyléthyle, un groupe propoxycarbonylpropyle, un groupe dioxolanylméthyle, un groupe dioxolanyléthyle, un groupe dioxanylméthyle, un groupe dioxanyléthyle, un groupe oxolanylméthyle, un groupe oxolanyléthyle, un groupe cyclopropylméthyle, un groupe dichlorocyclopropylméthyle, un groupe diméthylcyclopropylméthyle, un groupe dichlorodiméthylcyclopropylméthyle, un groupe cyclopentyle, un groupe cyclohexyle ou un groupe cyclohexylméthyle ou

$R^1$ et $R^2$ représentent, conjointement avec l'atome d'azote auquel ils sont liés, un hétérocycle éventuellement substitué une à trois fois, de manière identique ou différente, répondant aux formules

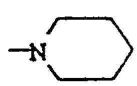 ; 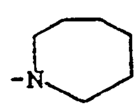 ; 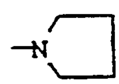 ou 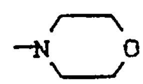

dans lesquelles, comme substituants, on envisage un groupe méthyle, un groupe éthyle, un groupe hydroxyméthyle, ainsi que leurs sels d'addition d'acides,

leurs isomères géométriques et optiques, ainsi que leurs mélanges d'isomères.

**4.** Procédé pour la préparation de composés hétérocycliques aminométhylés répondant à la formule générale (I)

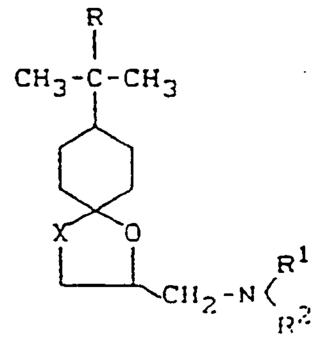

(I)

dans laquelle

X représente un atome d'oxygène ou un atome de soufre,

R représente un atome d'hydrogène, un groupe alkyle à chaîne droite ou ramifiée et contenant de 1 à 6 atomes de carbone ou un groupe phényle ou cyclohexyle, chaque fois éventuellement substitué de une à trois fois, de manière identique ou différente, par un groupe alkyle contenant de l à 4 atomes de carbone et/ou par un atome d'halogène et

$R^1$ et $R^2$ représentent chacun, indépendamment l'un de l'autre, un atome d'hydrogène ; un groupe alkyle contenant de 1 à 12 atomes de carbone, un groupe alcényle contenant de 3 à 8 atomes de carbone, un groupe alcynyle contenant de 3 à 8 atomes de carbone, un groupe hydroxyalkyle contenant de 2 à 6 atomes de carbone, un groupe alcoxyalkyle ou dialcoxyalkyle contenant chacun de 1 à 6 atomes de carbone ou un groupe hydroxyalcoxyalkyle contenant de 2 à 6 atomes de carbone dans les fractions alkyle individuelles, ces groupes étant chacun à chaîne droite ou ramifiée ; un groupe alcoxycarbonylalkyle contenant de 1 à 6 atomes de carbone, chaque fois, dans la fraction alcoxy ou alkyle, un groupe dioxolanylalkyle, un groupe dioxanylalkyle ou un groupe oxolanylalkyle, chaque fois à chaîne droite ou ramifiée, contenant chacun de 1 à 4 atomes de carbone dans la fraction alkyle, ou un groupe cycloalkyle ou cycloalkylalkyle, chaque fois éventuellement substitué de manière identique ou différente, de une à plusieurs fois, dans la fraction alkyle, contenant chacun de 3 à 7 atomes de carbone dans la fraction cycloalkyle et éventuellement 1 à 4 atomes de carbone dans la fraction alkyle à chaîne droite ou ramifiée, groupes dans lesquels, comme substituants, on peut chaque fois envisager :

un atome d'halogène, un groupe alkyle, un groupe alcoxy, un groupe halogénalkyle ou un groupe halogénalcoxy, chaque fois à chaîne droite ou ramifiée, contenant de 1 à 4 atomes de carbone et éventuellement 1 à 9 atomes d'halogène identiques ou différents ; en outre, un groupe arylalkyle, un groupe arylalcényle ou un groupe aryle, chaque fois éventuellement substitué une à plusieurs fois, de manière identique ou différente, dans la fraction aryle, contenant chacun de 6 à 10 atomes de carbone dans la fraction aryle et éventuellement jusqu'à 6 atomes de carbone dans la fraction alkyle ou alcényle à chaîne droite ou ramifiée ; groupes dans lesquels, comme substituants du groupe aryle, on peut chaque fois envisager :

un atome d'halogène, un groupe cyano, un groupe nitro, un groupe alkyle, un groupe alcoxy, un groupe alkylthio, un groupe halogénalkyle, un groupe halogénalcoxy, un groupe halogénalkylthio, un groupe alcoxycarbonyle ou un groupe alcoximinoalkyle chaque fois à chaîne droite ou ramifiée, contenant chacun de 1 à 4 atomes de carbone dans les fractions alkyle individuelles et éventuellement 1 à 9 atomes d'halogène identiques ou différents ou

$R^1$ et $R^2$ représentent, conjointement avec l'atome d'azote auquel ils sont liés, un hétérocycle penta- à heptagonal saturé, éventuellement substitué de une à plusieurs fois de manière identique ou différente, qui peut contenir éventuellement un autre hétéroatome, en particulier un atome d'azote, un atome d'oxygène ou un atome de soufre, dans lequel, comme substituants, on envisage : un groupe alkyle ou hydroxyalkyle chaque fois à chaîne droite ou ramifiée, contenant chacun de 1 à 4 atomes de carbone,

ainsi que leurs sels d'addition d'acides, leurs isomères géométriques et optiques, ainsi que leurs mélanges d'isomères, **caractérisé en ce qu'**on fait réagir

(a) des composés hétérocycliques substitués de formule (II)

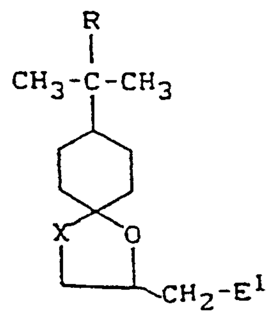

(II)

dans laquelle

R et X ont la signification mentionnée ci-dessus et

$E^1$ représente un groupe qui se sépare attirant les électrons,

avec des amines de formule (III)

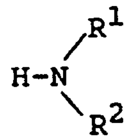

dans lesquelles

$R^1$ et $R^2$ ont la signification mentionnée ci-dessus,

éventuellement en présence d'un diluant et éventuellement en présence d'un fixateur d'acides, ou

(b) on fait réagir les composés hétérocycliques aminométhylés, que l'on peut obtenir par le procédé (a) et qui répondent à la formule (Ia)

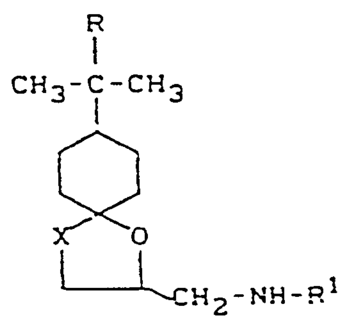

(Ia)

dans laquelle R, $R^1$ et X ont la signification mentionnée ci-dessus,

avec des agents d'alkylation de formule (IV)

$R^{2-1}$-$E^2$ (IV)

dans laquelle

$R^{2-1}$ représente un groupe alkyle contenant de 1 à 12 atomes de carbone, un groupe alcényle contenant de 3 à 8 atomes de carbone, un groupe alcynyle contenant de 3 à 8 atomes de carbone, un groupe hydroxyalkyle contenant de 2 à 6 atomes de carbone, un groupe alcoxyalkyle ou dialcoxyalkyle contenant chacun de 1 à 6 atomes de carbone ou un groupe hydroxyalcoxyalkyle contenant de 2 à 6 atomes de carbone dans les fractions

alkyle individuelles, ces groupes étant chacun à chaîne droite ou ramifiée ; un groupe alcoxycarbonylalkyle contenant de 1 à 6 atomes de carbone, chaque fois, dans la fraction alcoxy ou alkyle, un groupe dioxolanylalkyle, un groupe dioxanylalkyle ou un groupe oxolanylalkyle, chaque fois à chaîne droite ou ramifiée, contenant chacun de l à 4 atomes de carbone dans la fraction alkyle ; un groupe cycloalkyle ou cycloalkylalkyle, chaque fois éventuellement substitué, de manière identique ou différente, de une à plusieurs fois, dans la fraction alkyle, contenant chacun de 3 à 7 atomes de carbone dans la fraction cycloalkyle et éventuellement 1 à 4 atomes de carbone dans la fraction alkyle à chaîne droite ou ramifiée, groupes dans lesquels, comme substituants, on peut chaque fois envisager : un atome d'halogène, un groupe alkyle, un groupe alcoxy, un groupe halogénalkyle ou un groupe halogénalcoxy, chaque fois à chaîne droite ou ramifiée, contenant de 1 à 4 atomes de carbone et éventuellement 1 à 9 atomes d'halogène identiques ou différents ; un groupe arylalkyle ou un groupe arylalcényle, chaque fois éventuellement substitué une à plusieurs fois, de manière identique ou différente dans la fraction aryle, contenant chacun de 6 à 10 atomes de carbone dans la fraction aryle et jusqu'à 6 atomes de carbone dans la fraction alkyle ou alcényle à chaîne droite ou ramifiée, dans lequel, comme substituants du groupe aryle, on peut chaque fois envisager : un atome d'halogène, un groupe cyano, un groupe nitro, un groupe alkyle, un groupe alcoxy, un groupe alkylthio, un groupe halogénalkyle, un groupe halogénalcoxy, un groupe halogénalkylthio, un groupe alcoxycarbonyle ou un groupe alcoximinoalkyle chaque fois à chaîne droite ou ramifiée, contenant chacun de 1 à 4 atomes de carbone dans les fractions alkyle individuelles et éventuellement 1 à 9 atomes d'halogène identiques ou différents

et

$E^2$ représente un groupe qui se sépare attirant les électrons,

éventuellement en présence d'un diluant et éventuellement en présence d'un fixateur d'acide et ensuite, on ajoute éventuellement un acide ou encore on met en oeuvre un procédé physique de séparation.

5. Agent pour la lutte contre les parasites, **caractérisé en ce qu'**il contient au moins un composé hétérocyclique aminométhylé de formule (I), selon les revendications 1 et 4.

6. Utilisation de composés hétérocycliques aminométhylés de formule (I) selon les revendications 1 et 4, pour lutter contre les parasites.

7. Procédé pour la lutte contre les parasites, **caractérisé en ce qu'**on laisse agir des composés hétérocycliques aminométhylés de formule (I), selon les revendications 1 et 4, sur des parasites et/ou dans leur biotope.

8. Procédé pour la préparation d'agents pour la lutte contre les parasites, **caractérisé en ce qu'**on mélange des composés hétérocycliques aminométhylés de formule (I), selon les revendications 1 et 4, avec des diluants et/ou des agents tensio-actifs.